# EUROPEAN PATENT SPECIFICATION

(11) **EP 4 522 075 B1**
(45) Date of publication and mention of the grant of the patent: **16.09.2026**
(21) Application number: 23724111.2
(22) Date of filing: 20.04.2023
(51) Int. Cl.: A61F 2/24

(54) **TRANSCATHETER VALVE - SINGLE STENT STRUCTURE WITH FABRIC**
TRANSKATHETERKLAPPE - EINZELSTENTSTRUKTUR MIT GEWEBE
STRUCTURE DE VALVE TRANSCATHÉTER-STENT UNIQUE AVEC TISSU

(30) Priority: 13.05.2022 US 202263341702 P
(43) Date of publication of application: 19.03.2025
(73) Proprietor: St. Jude Medical, Cardiology Division, Inc., St. Paul MN 55117 (US)
(72) Inventor: PECKELS, William, Robbinsdale, Minnesota 55422 (US); MARNACH, Heath, Minneapolis, Minnesota 55419 (US); HUDDLESTON, Preston, Maplewood, Minnesota 55117 (US); MAI, Son, North Branch, Minnesota 55056 (US); VIDLUND, Zachary, Robbinsdale, Minnesota 55422 (US); KING, Alec, Maple Grove, Minnesota 55369 (US)
(74) Representative: Gill Jennings & Every LLP
(86) International application number: PCT/US2023/019241
(87) International publication number: WO 2023/219771

(56) References cited:
- WO-A1-2022/047395
- US-A1- 2014 277 390
- US-A1- 2018 125 648

## Description

### CROSS-REFERENCE TO RELATED APPLICATIONS

This application claims priority to U.S. Provisional Patent Application No. 63/341,702, filed May 13, 2022.

### FIELD AND BACKGROUND OF THE DISCLOSURE

Heart valve disease is a significant cause of morbidity and mortality. One treatment for this disease is valve replacement. One form of replacement device is a bioprosthetic valve. Collapsing these valves to a smaller size or into a delivery system enables less invasive delivery approaches compared to conventional open-chest, open-heart surgery. Collapsing the implant to a smaller size and using a smaller delivery system minimizes the access site size and reduces the number of potential periprocedural complications.

The size to which an implant can be collapsed is limited by the volume of materials used in the implant, the strengths and shapes of those materials, and the need to function after re-expansion. Using multiple steps and/or multiple delivery system devices may increase the time and complexity of a procedure.

Native atrioventricular valves (*i.e.,* the tricuspid valve and the mitral valve) typically have a larger size and/or diameter compared to the native aortic valve and the native pulmonary valve. Among the native atrioventricular valves, a regurgitant tricuspid valve typically has a larger size and/or diameter than a regurgitant mitral valve. For example, for patients with severe tricuspid valve regurgitation, the diameter of the tricuspid valve may range from about 40 mm to about 66 mm, although these numbers are merely exemplary. As a result, prosthetic heart valve designs and considerations for replacing the different native heart valves are not identical. For example, to accommodate the large size of the mitral and tricuspid valve, recent prosthetic heart valve designs have included an outer frame with a large size to engage the native mitral or tricuspid annulus, and a smaller and generally cylindrical inner frame within that outer frame, the inner frame housing the prosthetic valve leaflets. However, this double-stented design generally increases the bulk of the prosthetic heart valve, resulting in a larger profile when collapsed within a delivery device. This, in turn, requires the delivery device (*e.g.,* a catheter housing the collapsed prosthetic heart valve for delivery) to have a larger size to accommodate the large prosthetic heart valve. Typically, it is desirable for catheters of transcatheter heart valve delivery devices to have a smaller size, since the catheters may need to pass through the vasculature to reach the native heart valve in a minimally invasive manner. Thus, it would be desirable to have a prosthetic heart valve that is able to fit within a native tricuspid or mitral valve but is able to collapse to a small size to be accommodated in a relatively small profile delivery device.

US 2018/125648 A1 relates to a heart valve system including a radially self-expandable tubular body. WO 2022/047395 A1 relates to a device for treating a diseased native valve including a frame structure having an unexpanded configuration and an expanded configuration. US 2014/277390 A1 relates to expandable prostheses such as replacement heart valves, that are configured to atraumatically grasp intralumenal tissue.

### BRIEF SUMMARY

According to one aspect of the disclosure, a prosthetic heart valve is for replacing a native atrioventricular valve. The prosthetic heart valve includes a self-expanding stent formed as a monolithic structure, the stent including an atrial disk, a ventricular disk, and a center portion extending between the atrial disk and the ventricular disk. A plurality of prosthetic leaflets is directly coupled to the center portion of the stent. An outer fabric is coupled to the stent. In an implanted condition of the prosthetic heart valve, the atrial disk is sized to contact an atrial side of the native atrioventricular valve, the ventricular disk is sized to contact a ventricular side of the native atrioventricular valve, the center portion is sized to be positioned radially inside the native atrioventricular valve without pressing against the native atrioventricular valve, and the outer fabric is configured to directly contact the native atrioventricular valve. In a collapsed condition of the prosthetic heart valve, the prosthetic heart valve may be sized to be received within a delivery catheter for delivery to the patient, the delivery catheter having an inner diameter of between 22 French and 32 French, or between 24 French and 28 French. In an expanded condition of the stent, the central portion has a diameter, and the ventricular disk may have a diameter that is between 1.5 and 2.5 times larger than the diameter of the central portion, or between 2.0 and 2.25 times larger than the diameter of the central portion.

The outer fabric includes a first outer fabric and a second outer fabric, the first outer fabric being formed as an extensible fabric (for example, a knitted fabric) that is not directly coupled to the central portion of the stent, the second outer fabric being formed as a non-extensible fabric (for example, a woven fabric), the second outer fabric including a first portion directly coupled to the atrial disk and a second portion directly coupled to the ventricular disk. A first end of the first outer fabric may be coupled to the first portion of the second outer fabric at a first seam, and a second end of the first outer fabric may be coupled to the second portion of the second outer fabric at a second seam. The first seam and the second seam may be formed via sutures or ultrasonic welding. As the prosthetic heart valve transitions from an expanded condition to a collapsed condition, the first outer fabric may stretch to increase in length by a factor of greater than 1 and less than 3.

The outer fabric may include a single outer fabric that is woven, a first end of the outer fabric being directly coupled to the atrial disk, and a second end of the fabric being directly coupled to the ventricular disk, the outer fabric including a central portion forming at least one pleat. As the prosthetic heart valve transitions from an expanded condition to a collapsed condition, the at least one pleat may unfold so that the outer fabric does not prevent the prosthetic heart valve from transitioning from the expanded condition to the collapsed condition. At least one suture may extend radially between the outer fabric and the stent, a first end of the at least one suture being coupled to the outer fabric, a second end of the at least one suture being coupled to the stent.

The stent may include a plurality of cells, selected ones of the cells having a free end hooking radially outwardly adjacent the center portion of the stent in an expanded condition of the stent, the free end configured to press the outer fabric radially outwardly. Alternatively, selected ones of the cells may have a protrusion extending radially outwardly adjacent the center portion of the stent in an expanded condition of the stent, the protrusion configured to press the outer fabric radially outwardly.

The ventricular disk may include a plurality of diamond-shaped ventricular cells that extend substantially parallel to a center longitudinal axis of the stent in an expanded condition of the stent. Some of the plurality of diamond-shaped ventricular cells may include a sharp tip configured to pierce tissue of the native atrioventricular valve when the prosthetic heart valve is in the implanted condition. Some of the plurality of diamond-shaped ventricular cells may include blunt tips configured to avoid piercing tissue of the native atrioventricular valve when the prosthetic heart valve is in the implanted condition.

In some embodiments, the ventricular disk includes diamond-shaped ventricular cells that each include a tine, each tine having a first end coupled to an outflow apex of a corresponding one of the ventricular cells and an opposite free end, each the tine extending radially outwardly relative to the corresponding one of the ventricular cells at an angle of between 10 and 20 degrees. In other embodiments, the stent includes a plurality of vertical struts extending from the atrial disk to the ventricular disk, and the ventricular disk includes a plurality of "V"-shaped struts extending circumferentially between adjacent ones of the vertical struts, a pointed end of the "V"-shaped struts flaring radially outwardly from the ventricular disk in the expanded condition of the stent.

### BRIEF DESCRIPTION OF DRAWINGS

Fig. 1 is a cross-section of a prosthetic heart valve, taken along section line 1-1 of Fig. 2.
Fig. 2 is a schematic side view of the prosthetic heart valve of Fig. 1.
Fig. 3 is a side view of the prosthetic heart valve of Figs. 1-2.
Fig. 4 is a top or atrial view of the prosthetic heart valve of Figs. 1-2.
Fig. 5 is a bottom or ventricular view of the prosthetic heart valve of Figs. 1-2.
Fig. 6 is a perspective view of the stent of the prosthetic heart valve of Figs. 1-2 with other components of the prosthetic heart valve omitted.
Fig. 7 is a view of a portion of the cut pattern of the stent of Fig. 6.
Fig. 8 is a perspective view of another embodiment of a stent for use with a prosthetic heart valve like that of Figs. 1-2.
Fig. 9 is a view of a portion of the cut pattern of the stent of Fig. 8.
Fig. 10 is a view of a portion of the cut pattern of an alternative version of the stent of Fig. 8.
Fig. 11 is a view of the cut pattern of Fig. 10 with the stent being slightly expanded to illustrate the stent structure more clearly.
Fig. 12A is a perspective view of a stent similar to that shown in Fig. 6, according to another aspect of the disclosure.
Fig. 12B is a perspective view of an alternate version of the stent of Fig. 12A.
Fig. 13 is a perspective view of another embodiment of a stent for use with a prosthetic heart valve like that of Figs. 1-2.
Fig. 14 is a view of a portion of the cut pattern of the stent of Fig. 13.
Figs. 15-16 are side and perspective views, respectively, of a prosthetic heart valve according to another aspect of the disclosure.
Fig. 17 is a highly schematic drawing of a stent of a prosthetic heart valve deployed within a native tricuspid valve.
Fig. 18 is a highly schematic drawing of tips of certain cells of the prosthetic heart valve stent of Fig. 17.
Fig. 19 is a highly schematic drawing of a stent of a prosthetic heart valve deployed within a native tricuspid valve.
Figs. 20-21 are highly schematic drawings of front and side views of ventricular tips of the stent of Fig. 19.
Fig. 22 is a highly schematic drawing of a prosthetic heart valve deployed within a native tricuspid valve.
Fig. 23 is a highly schematic drawing of a prosthetic heart valve in an expanded condition.
Fig. 24 is an enlarged view of ventricular cells of the prosthetic heart valve of Fig. 23.
Fig. 25 is a perspective view of the stent of Fig. 6 with a commissure splay control feature.
Fig. 26 is a schematic bottom view of the stent of Fig. 6 with the commissure splay control feature of Fig. 25 and a commissure deflection control feature.
Fig. 27 is a highly schematic cross-section of a double-frame prosthetic heart valve with an outer fabric in an expanded condition.
Fig. 28 is a highly schematic cross-section of the prosthetic heart valve of Fig. 27 in a collapsed condition.
Fig. 29 is a highly schematic cross-section of a double-frame prosthetic heart valve with an alternate version of an outer fabric to that shown in Fig. 27, in an expanded condition.
Fig. 30 is a highly schematic cross-section of the prosthetic heart valve of Fig. 29 in a collapsed condition.
Fig. 31 is a highly schematic cross-section of a double-frame prosthetic heart valve with an alternate version of an outer fabric to those shown in Figs. 27 and 29, in an expanded condition.
Fig. 32 is a highly schematic cross-section of the prosthetic heart valve of Fig. 21 in a collapsed condition.

### DETAILED DESCRIPTION OF THE DISCLOSURE

As used herein, the term inflow, when used in connection with a prosthetic heart valve, refers to the end of the prosthetic heart valve through which blood first flows when flowing in the antegrade direction, and the term outflow refers to the end of the prosthetic heart valve through which blood last flows when flowing in the antegrade direction. Further, although the disclosure focuses on prosthetic tricuspid valve replacements, the disclosure applies with similar force to prosthetic mitral valve replacements. Thus, unless otherwise expressly specified, the embodiments described herein may be used for replacing either a native tricuspid valve or a native mitral valve (with or without additional modifications specific to the heart valve being replaced), even if a particular embodiment may be more suited for replacing either the native tricuspid valve or the native mitral valve.

As explained in the background of the disclosure, prosthetic heart valves that include an anchoring frame and a valve frame nested within the anchoring frame typically have larger sizes when collapsed within a delivery device. As an example, this type of prosthetic heart valve may only fit within a delivery device that has a catheter with an inner diameter that is 30 French (10 mm in diameter) or larger. For transcatheter prosthetic mitral or tricuspid valves that are delivered intravascularly through the femoral artery, a delivery catheter having an outer diameter of 30 French or larger may increase the likelihood of access site complications, which may require a surgeon to intervene. The prosthetic heart valves disclosed herein have features and configurations that are intended to allow for the prosthetic heart valves to have a large enough footprint to reliably anchor within the larger size annulus of the tricuspid valve (or the mitral valve) while being able to collapse into a delivery catheter having an inner diameter of 30 French or smaller. It should be understood that, as used herein, the unit French refers to the inner diameter of a catheter when describing the ability of a valve to fit within that catheter, whereas the unit French refers to the outer diameter of the catheter when describing how catheter size may result in vascular access problems.

As is described below, one way to achieve this functionality is to design the prosthetic heart valve with a single support stent *(e.g.,* a single stent layer or a non-nested frame configuration) that can span the large atrioventricular valve annulus diameters found in patients who experience heart failure. The geometry of the support stent may allow for prosthetic leaflets to be secured inside, with atrial and/or ventricular flanges or disks that have a large enough diameter or profile to clamp the native annulus tissue therebetween. To provide adequate sealing between the support stent and the native valve annulus, fabric(s) may span the gap between the atrial and ventricular disks, where the fabric(s) are capable of elongating to mitigate the effects of foreshortening when sheathing the prosthetic heart valve into the catheter. While the embodiments described below may be suitable for replacing either a tricuspid or mitral valve, these embodiments may be best suited for replacing a tricuspid valve due to the lower right ventricular pressures, compared to left ventricular pressures, which may reduce the need for a nested stent design.

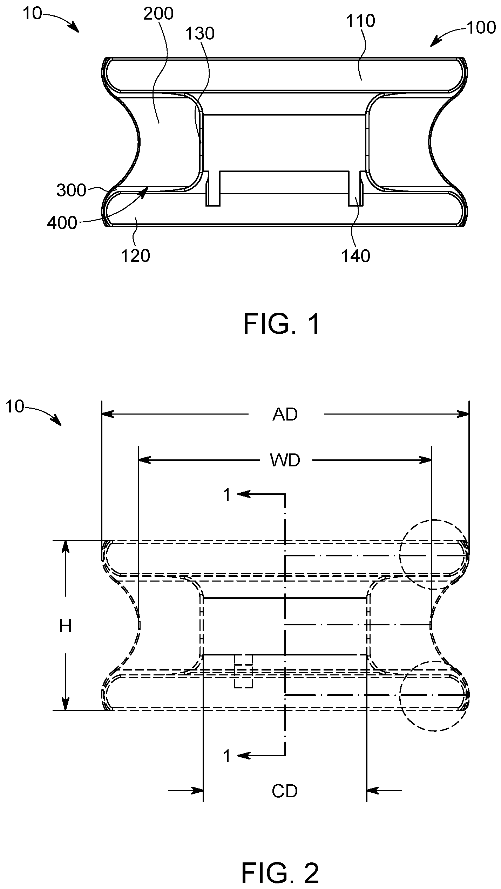

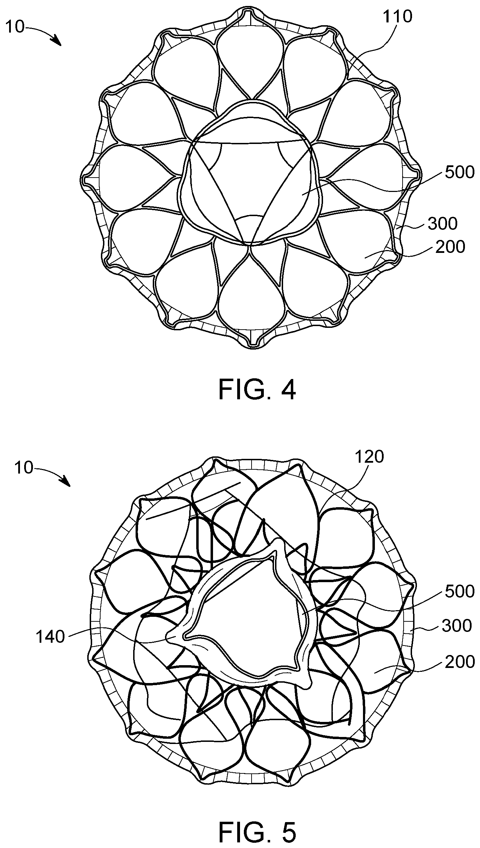

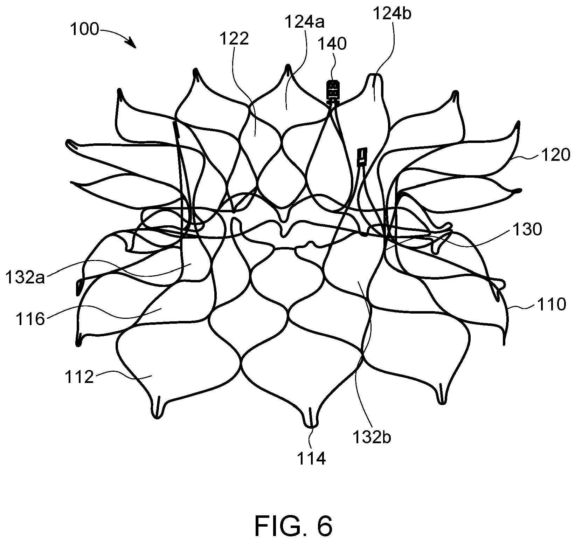

Fig. 1 is a cross-section of a prosthetic heart valve 10, taken along section line 1-1 of Fig. 2, according to one aspect of the disclosure. Generally, prosthetic heart valve 10 includes a support frame or stent 100, which may include an atrial flange 110 (which may alternately be referred to as an atrial disk or anchor), a ventricular flange 120 (which may alternately be referred to as a ventricular disk or anchor), and a central stent portion 130. The stent 100 may also include a plurality of commissure attachment features ("CAFs") 140 for use in coupling the prosthetic valve leaflets 500 to the stent 100. The prosthetic valve leaflets 500 are omitted from the illustration of Figs. 1-2 and are best shown in Figs. 4-5. The stent 100 is described in greater detail below in connection with Figs. 6-7. Referring still to Fig. 1, the prosthetic heart valve 10 may include a first fabric 200 generally surrounding the central portion 130 of the stent 100 and spanning the gap between the atrial disk 110 and the ventricular disk 120. In one embodiment, the first fabric may be formed as a knitted fabric (*e.g*., polyethylene terephthalate ("PET"), polytetrafluoroethylene ("PTFE"), ultra-high molecular weight polyethylene ("UHMWPE"), polyester, or similar materials). The formation of the first fabric 200 as a knitted fabric may allow for significant stretching of the first fabric, *e.g.,* up to doubling or tripling in length upon being stretched. In one embodiment, the first fabric 200 is configured to stretch to increase its length by a factor of about 2.5. When in the expanded or deployed condition shown in Fig. 1, a substantial amount of open space or volume may exist between the first fabric 200 and the outside of the stent 100, at least prior to implantation. A second fabric 300 may be provided on the outer surface of the atrial disk 110 and the ventricular disk 120, the second fabric 300 being coupled to the first fabric 200 at seams 400, which may be for example ultrasonic welding seams. In one embodiment, the second fabric 300 may be formed as a woven fabric (*e.g.,* PET, PTFE, UHMWPE, *etc.*)*.* By forming the second fabric 300 as a woven fabric, the second fabric 300 may be particularly suited to provide sealing functionality against the native anatomy, while the knitted fabric 200 is particularly suited to provide stretching capabilities to allow for relatively unhindered collapsing and expanding of the stent 100. Even if the first fabric 200 is formed as a knit fabric and the second fabric 300 is formed as a woven fabric, it may be desirable (although not required) for the two fabrics to be formed of the same material, with the stretch and/or sealing properties being influenced, at least in part, by the way in which the threads of the fabric are wound, knitted, and/or woven. For example, changing the bias of the warp versus the weft will change the stretch capabilities of a fabric.

Referring briefly to Fig. 2, various exemplary dimensions of the prosthetic heart valve 10 when the prosthetic heart valve 10 is in an expanded or deployed configuration are provided. However, these dimensions are merely exemplary and other dimensions may be suitable. For example, the outer anchor diameter AD of the atrial disk 110 and/or ventricular disk 120 may be about 65 mm. However, in other embodiments, the anchor diameter AD may be between about 50 mm and about 80 mm. In the expanded or deployed condition (e.g., upon implantation into the native tricuspid valve annulus), the first fabric 200 may form a waisted shape with a minimum waist diameter WD near an axial center of the first fabric 200. In the illustrated embodiment, the waist diameter WD may be about 52 mm, but in other embodiments, the waist diameter WD may be between about 45 mm and about 75 mm. The center portion 130 of the stent 100 may be generally cylindrical, and in the illustrated embodiment has a central diameter CD of about 29 mm, but in other embodiments, the central diameter CD may be between about 25 mm and about 35 mm. The prosthetic heart valve 10 may have a height H between the inflow and outflow ends of about 30 mm, but in other embodiments, the height may be between about 25 mm and about 45 mm. As noted above, each of these dimensions is merely illustrative. In some embodiments, in the expanded or deployed condition, the ratio of the outer anchor diameter AD to the central diameter CD is between about 2.5:1 and about 1.5:1, preferably between about 2.5:1 and about 2.0:1, including about 2.25:1. As is explained in greater detail below, this large disparity may allow the atrial disk 110 and ventricular disk 120 to be large enough to provide anchoring, but the central portion 130 to be small enough to house an appropriately sized set of prosthetic valve leaflets 500, while still maintaining a small crimp profile for delivery, *e.g*. capable of being delivered within, and deployed successfully from, a delivery device catheter having an inner diameter as small as 24 French (8.0 mm) or even as small as 18 French (6.0 mm). In some embodiments, the prosthetic heart valve may be successfully housed within a catheter having an inner diameter of between 22 French (7.33 mm) and 32 French (10.66 mm), including between about 24 French (8.0mm) and 28 French (9.33 mm).

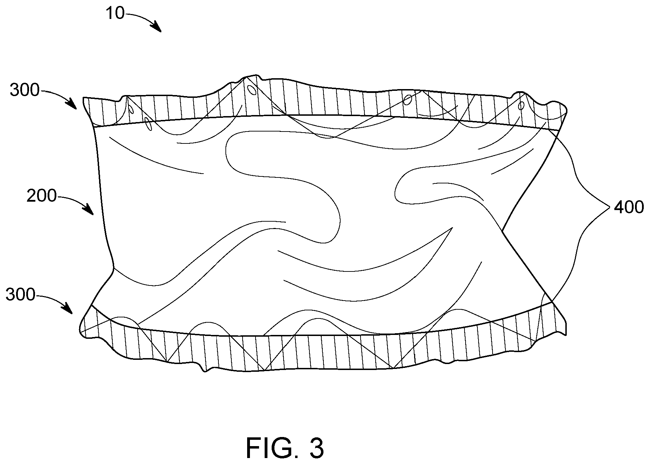

Fig. 3 is a side view of the prosthetic heart valve 10. Fig. 3 illustrates the prosthetic heart valve 10 in an expanded or deployed condition, with the first fabric 200 extending between the atrial disk 110 and the ventricular disk 120. In the illustrated embodiment, first fabric 200 is a stretchy fabric that can easily elongate when the prosthetic heart valve 10 collapses and foreshorten as the prosthetic heart valve 10 expands. To achieve this elongation and foreshortening, it is preferred that the first fabric 200 is not directly coupled to the stent 100. For example, the fabric 200 preferably is not directly sutured to struts of the stent 100. The second fabric 300 includes a portion coupled to the outside of the atrial disk 110 and a portion coupled to the outside of the ventricular disk 110, for example by suturing. As noted above, the second fabric 300 may function to assist with creating or enhancing the seal between the native valve annulus and the atrial and ventricular portions of the prosthetic heart valve 10. The interfaces 400 between the first fabric 200 and the portions of the second fabric 300 are shown in Fig. 3. In the illustrated example, the interfaces 400 are seams created via ultrasonic welding, but the fabrics may be coupled to each other in any suitable way, including suturing, adhesives, *etc.* In some embodiments, only a single outer fabric may be used, with the single outer fabric having sufficient stretch capabilities to allow the stent 100 to expand and collapse without significant restriction, but while also providing suitable sealing with the native valve annulus.

Fig. 4 illustrates the prosthetic heart valve 10 in an expanded or deployed condition, as viewed from the atrial or inflow side with the prosthetic leaflets 500 in an open condition. Fig. 5 illustrates the prosthetic heart valve 10 in an expanded or deployed condition, as viewed from the ventricular or outflow side with the prosthetic leaflets 500 in an open condition. In the illustrated embodiment, the prosthetic heart valve 10 includes three prosthetic leaflets 500, but in some embodiments, the prosthetic heart valve 10 may include more or fewer prosthetic leaflets. The prosthetic leaflets 500 may be formed of any suitable material. For example, each prosthetic leaflet 500 may be formed of bioprosthetic tissue, such as porcine or bovine pericardium. In other embodiments, each prosthetic leaflet 500 may be formed of a synthetic material such as a synthetic material or fabric, including PET, UHMWPE, PTFE, *etc.* Each prosthetic leaflet 500 may have two side portions (*e.g.,* forming leaflet tabs), each side portion being coupled to an end of an adjacent prosthetic leaflet 500 to form a commissure, each leaflet commissure being coupled to a respective CAF 140 of the stent 100. Each leaflet may include an outflow edge between the two side edges, the outflow edge being free to allow for opening and closing of the leaflets during normal operation (*e.g.*, to provide the valve functionality). Each leaflet may include an inflow edge between the two side edges, the inflow edge being coupled (*e.g.*, sutured) to the stent 100. In some embodiments, the inflow edges of the prosthetic leaflets 500 are coupled solely (*e.g.,* via sutures) to struts of the central portion 130 of the stent 100, without any direct attachment to other components. In other embodiments, a short fabric skirt may be provided around a portion of the inflow side of the central portion 130 of the stent 100, with the short fabric skirt coupled to the stent 100 and providing an additional structure that may be used for coupling (*e.g.*, via sutures) the inflow ends of the prosthetic leaflets 500 to the prosthetic heart valve 10.

By comparing Fig. 4 to Fig. 5, it can be seen that the atrial disk 110 is "closed," while the ventricular disk 120 is "open." In other words, as shown in Fig. 4, the inflow ends of the prosthetic leaflets 500 are directly coupled to the stent 100 (and/or a separate short fabric skirt as noted above) so that there is no open flow pathway between the prosthetic leaflets 500 and the outer fabric skirt(s) (*e.g*., first fabric 200 and second fabric 300, or the single outer fabric if only a single outer fabric is used) at the atrial disk 110. On the other hand, as shown in Fig. 5, the outflow ends of the prosthetic leaflets 500 have a significant radial spacing from the first fabric 200 and the second fabric 300. With this configuration, after implantation of the prosthetic heart valve 10 into the native tricuspid valve, as the right ventricle contracts, the prosthetic leaflets 500 are forced closed and blood tends to flow in the retrograde direction into the space between the prosthetic leaflets 500 and the first fabric 200. The "closed" configuration at the atrial disk 110 ensures that blood cannot actually pass into the right atrium, but the "open" configuration at the ventricular disk 120 allows that retrograde blood to press the first fabric 200 outwardly and into the native valve annulus to enhance the seal between the prosthetic heart valve 10 and the native tricuspid valve annulus.

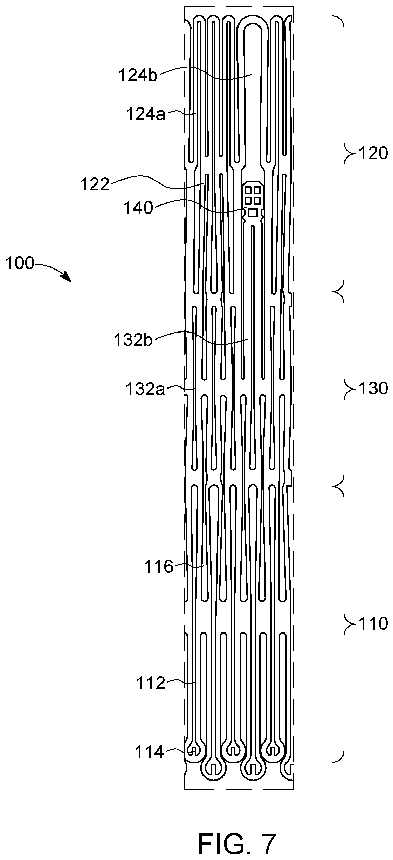

Fig. 6 is a perspective view of the stent 100 of the prosthetic heart valve 10 in an expanded condition, with other components of the prosthetic heart valve 10 omitted from the figure. The orientation of stent 100 is opposite that shown in Figs. 1-3. In other words, the top of the view of Fig. 6 is the outflow end and the bottom of the view of Fig. 6 is the inflow end. Stent 100 is preferably formed of a biocompatible shape memory or superelastic material. One particularly suitable example of this stent material is a nickel-titanium alloy, such as nitinol. However, other materials may be suitable. In one example, stent 100 may be formed by laser cutting a hollow tube of nitinol, and then shape-set to the desired shape, for example by treatment. With this configuration, the stent 100 may take the set shape, such as that shown in Fig. 6, in the absence of applied forces. To deliver the prosthetic heart valve 10, the prosthetic heart valve 10 may be collapsed to a small diameter and positioned within a delivery catheter to be passed intravascularly through the patient into the patient's heart. Fig. 7 shows the cut pattern of a portion of stent 100.

Referring collectively to Figs. 6-7, the stent 100 may be formed with a plurality of rows of generally diamond-shaped cells. In the illustrated example, the atrial disk 110 includes an inflow row of cells 112, which may include a total of twelve cells. A tine or pin 114 may be formed at the inflow apex of each cell 112, the pin extending a short distance in the outflow direction to a free end. Each pin 114 may be sized and shaped so that a suture loop of the delivery device may slip over the pin 114, keeping the stent 110 connected to the delivery device during delivery and deployment. Upon deployment of the prosthetic heart valve 10, each suture loop may be pushed forward or distally to disengage with the corresponding pins 114 to fully decouple the prosthetic heart valve 10 from the delivery device. Similar pins and suture loops are described in more detail in U.S. Patent No. 10,874,512.

A transition row of cells 116 may be positioned directly adjacent to the inflow row of cells 112, with cells 116 transitioning between the atrial disk 110 and the central portion 130. In the illustrated embodiment, a total of twelve atrial transition cells 116 are provided. The central portion 130 may be generally formed by a row of central cells, although each central cell may not be identical to each other central cell. In the illustrated example, there are a total of twelve central cells, with a total of nine connected central cells 132a, and three free central cells 132b. Each connected central cell 132a may be diamond-shaped and have both inflow and outflow apices of the central cell 132a connected directly to another cell of the stent. Each free central cell 132b may have an inflow apex coupled directly to the outflow apex of an inflow cell 112, with the outflow apex of the free central cell 132b transitioning into a CAF 140. With this configuration, there are three free central cells 132b evenly spaced around the circumference of the stent 100, and three consecutive connected central cells 132a positioned between each pair of free central cells 132b. In the illustrated embodiment, each CAF 140 is cantilevered in the sense that it is only coupled to the stent 100 on one side, via two struts of the corresponding free central cell 132b. This cantilevered configuration may allow for greater leaflet height while maintaining a relatively small distance between the atrial disk 110 and the ventricular disk 120. The cantilevered configuration may also allow for greater deflection of the CAFs 140 during normal operation of the prosthetic heart valve 10, which may reduce stress on the prosthetic leaflets 500. Each CAF 140 may include one or more eyelets to assist in suturing or otherwise coupling the commissures of the prosthetic leaflets 500 to the CAFs 140. Each CAF 140 may also include a separate fabric coupled to the CAF 140 to assist with suturing of the prosthetic leaflets 500 to the CAF 140. In the illustrated embodiment, each CAF 140 includes a two-by-two array of four small eyelets, with a larger eyelet positioned between the array of four small eyelets and the two struts of the free central cell 132b. However, other configurations of eyelets and other types of CAFs may be suitable as an alternative to CAF 140.

Still referring to Figs. 6-7, a row of ventricular transition cells 122 may be positioned directly adjacent to the central cells. In the illustrated embodiment, there are a total of six ventricular transition cells 122 provided in pairs, with each ventricular transition cell 122 positioned between two adjacent connected central cells 132. In this example, there are no ventricular transition cells 122 directly adjacent to the free central cells 132b. The ventricular transition cells 122 may form part of the cylindrical central portion 130, and flare outwardly to form part of the ventricular disk 120. In the illustrated embodiment, the final ventricular row of cells includes small ventricular cells 124a and large ventricular cells 124b. For example, a total of nine small ventricular cells 124a may be provided, each with an inflow apex directly connected to the outflow apex of a connected central cell 132a. Between each series (*e.g*., series of three) small ventricular cells 124a, a large ventricular cell 124b is positioned. Portions of the free central cells 132b, including the CAFs 140, may nest within the large ventricular cell 124b when the stent 100 is collapsed. Thus, in the illustrated embodiment, a total of three large ventricular cells 124b are provided. Although one specific configuration of cells is shown and described in connection with Figs. 6-7, it should be understood that other configurations of cells may provide for suitable functionality of the prosthetic heart valve 10. With the embodiment described above, the stent 100 may be formed as a single unitary or monolithic structure that, when expanded or deployed, has an hourglass-type shape with two opposite disks having a large diameter for anchoring on the atrial and ventricular sides of the native annulus, with a generally cylindrical center portion extending therebetween having a significantly smaller diameter than the disks. As is explained in greater detail below, the central portion 130 of the stent 100 may be positioned a spaced distance from the native annulus after implantation, which is generally in contrast to typical valves where there is stent structure pushing directly against the native annulus tissue after the prosthetic heart valve is implanted. Rather, as described below, the first fabric 200 directly contacts the native annulus tissue upon implantation, with space remaining between the first fabric 200 and the central portion 130 of the stent 100. By avoiding having two separate stents that overlap and increase the bulk of the prosthetic heart valve when collapsed into a delivery device, prosthetic heart valve 10 may be collapsed into a catheter for delivery, the catheter having an inner diameter of smaller than 30 French, including for example 28 French or 24 French or even smaller. Other benefits of this single stent design may include lower cost, and feasible retrieval of the prosthetic heart valve, as a connector between two nested stents might make retrievability more difficult compared to a single stent design.

Referring briefly back to Figs. 1-3, to allow for the stent 100 to collapse into the delivery device and then expand upon deployment, the outer fabric is preferably designed to not overly restrict the stent 100 from collapsing and expanding. For example, sealing fabrics provided on stents of prosthetic heart valves are frequently woven fabrics and are often tightly sutured or otherwise coupled to the stent so that the fabric does not have the ability to significantly stretch or deviate from its position relative to the stent. Further, these fabrics are often designed with sealing against blood flow as a primary fabric characteristic. On the contrary, prosthetic heart valve 10 has an outer fabric that is capable of significant stretching to accommodate the change in the shape of the stent 100 during transitioning between the collapsed and expanded conditions, with the fabric being spaced a significant distance from the central portion 130 when the prosthetic heart valve 10 is deployed. For example, referring to Fig. 1, when the prosthetic heart valve 10 is deployed into the native valve annulus, the atrial disk 110 and ventricular disk 120 may generally wrap around the atrial and ventricular sides of the native valve annulus, with the outer fabric pressing against the native valve annulus (e.g., via systolic pressure against the fabric on the outflow side), and the central portion 130 of the stent 100 generally centered within (but not directly pressing against) the tissue of the valve annulus. This configuration may allow the central portion 130 of the stent 100 to be a smaller size (*e.g*., about 28 mm to 32 mm) which may be optimal for hemodynamics, without needing to add additional stent structure to allow for proper sealing and/or anchoring in the much larger sized tricuspid valve annulus. This, in turn, may allow for the prosthetic heart valve 10 to collapse down to a small size for loading into a relatively small delivery device to minimize the likelihood of procedural complications (in particular vascular access complications) resulting from the size of the delivery device. The outer fabric may be formed as a single piece of knit fabric that allows for stretching in one direction, with the ability to seal against the native anatomy and for blood to quickly clot into the fabric to reduce the likelihood of paravalvular ("PV") leak past the prosthetic heart valve 10 after implantation. However, in other embodiments, such as shown in Fig. 1, the outer fabric may include a first fabric 200 that is formed of a material chosen for its ability to stretch (*e.g.* a knit fabric), and a second fabric 300 at the atrial disk 110 and ventricular disk 120 that is chosen for the sealing, ingrowth, and/or clotting properties (*e.g*. a woven fabric), with the first fabric 200 coupled to the second fabric 300 in any desired fashion that allows the stretching to occur.

In some embodiments, the outer fabric (whether provided as a single outer fabric or a multi-piece outer fabric) may be designed with a pore size that allows blood to flow through the pores of the fabric but restricts thrombi from passing through the pores of the fabric. Although it is generally counter-intuitive to intentionally allow blood to pass through the fabric of a prosthetic heart valve, resulting in inefficient sealing, it may be appropriate in some situations, particularly for the tricuspid valve. For example, a patient with torrential tricuspid regurgitation may have very weak right heart tissue that is acclimated to relatively small pressure changes during the beating of the heart. If a prosthetic tricuspid valve with full sealing were implanted in a patient with torrential tricuspid regurgitation, the immediate result would be a significantly larger pressure differential during the beating of the heart due to the effective sealing of the prosthetic tricuspid valve. This rapid change in pressure could actually damage the heart further. In some patients, it may be desirable to allow for an amount of intentional regurgitation (*e.g.*, through pores of the fabric of the prosthetic heart valve) to reduce the initial pressure change upon prosthetic valve implantation. Over time, blood will clot into the fabric, with thrombi unable to escape and cause problems for the patient. As the blood clots into the fabric, the prosthetic heart valve will become more effective in sealing since blood will only pass through the prosthetic leaflets after the fabric is clotted. Thus, with this embodiment, the right heart is allowed more time to acclimate to the new hemodynamics to reduce the initial stress of implanting a fully sealing and fully functional prosthetic heart valve. In any of the embodiments described here, any fabrics intended to contact the native tissue may be provided with functional coatings, such as fibronectin (*e.g.*, to enhance the tissue ingrowth process) or an anti-coagulation factor to intentionally allow blood to leak through the prosthetic heart valve fabric, at least for a short time, to provide time to acclimate to the new hemodynamics upon implantation of the prosthetic heart valve.

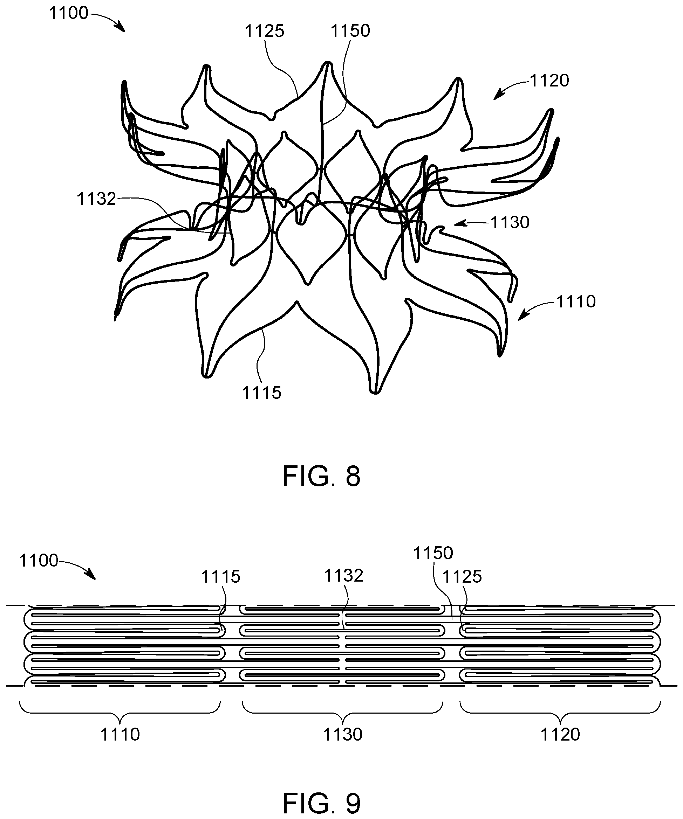

Fig. 8 is a perspective view of a stent 1100 according to another embodiment of the disclosure. The stent 1100 may be used with a prosthetic heart valve like that of prosthetic heart valve 10, with the main difference between replacing stent 100 with stent 1100. Fig. 9 illustrates a portion of a cut pattern of stent 1100. Stent 1100 may be formed of any of the materials described in connection with stent 100 and using any of the methods described in connection with stent 100. Because the only difference between stent 100 and stent 1100 is the stent pattern, only the stent pattern is described below. One of the main differences between stent 100 and stent 1100 is that stent 1100 includes a plurality of vertical struts 1150 that extend axially upon being cut. In this embodiment, stent 1100 includes a total of nine vertical struts 1150, each vertical strut 1150 extending the entire distance between the outflow end of the stent to the inflow end of the stent 1100.

Still referring to Figs. 8-9, the stent 1100 may include an atrial disk 1110, a ventricular disk 1120, and a center portion 1130, like that of stent 100. The atrial disk 1110 may include zigzag or inverted "V"-shape struts 1115 coupled between two adjacent vertical struts 1150, each of the "V"-shape struts 1115 being coupled to a terminal end of the respective vertical struts 1150 with a center portion pointing toward the center portion 1130 of the stent 1100. The ventricular disk 1120 may be formed similarly or identically to the atrial disk 1120, with "V"-shape struts 1125 being coupled to the opposite terminal ends of adjacent ones of the vertical struts 1150. The center portion 1130 of the stent 1100 may include a row of generally diamond-shaped center cells 1132, each center cell 1132 having free inflow and outflow apices, with the side apices being coupled to adjacent vertical struts 1150. With this configuration, the stent 1100 may be symmetric about a transverse plane through the axial center of the stent 1100. In other words, the stent 1100 may have the same shape when flipped upside down.

Like stent 100, stent 1100 may be shape set to have a generally hourglass-type shape, with an atrial disk 1110 and ventricular disk 1120 that extend a large distance radially outward from the center portion 1130, the center portion being generally cylindrical and formed mostly or entirely by the center cells 1132. When the stent 1100 transitions between the collapsed condition and the expanded condition, foreshortening may occur, but only because of the atrial disk 1110 and ventricular disk 1120 flaring radially outwardly. The vertical struts 1150 help ensure that no foreshortening occurs during stent expansion because of diamond-shaped cells "opening" from a collapsed condition to the diamond-shaped expanded condition. By limiting or eliminating the foreshortening during expansion, stent 1100 may be overall shorter when in the collapsed condition compared to a stent that undergoes foreshortening from diamond-cell expansion, such as stent 100. This, in turn, may allow for smaller axial space requirements within the delivery device to hold the stent 1100 (*e.g*., between 5 mm to 10 mm less than stent 100), which may be generally desirable. Stent 1100 is shown without any dedicated CAF features. The prosthetic leaflets may thus be coupled directly to struts of the stent 1100 in the absence of CAFs, or the stent 1100 may be designed with CAFs, described directly below. In some embodiments, stent 1100 could be used as a "docking station" by being implanted first, with a separate prosthetic heart valve (*e.g.*, a balloon-expandable or even self-expandable prosthetic heart valve) being implanted into the stent 1100.

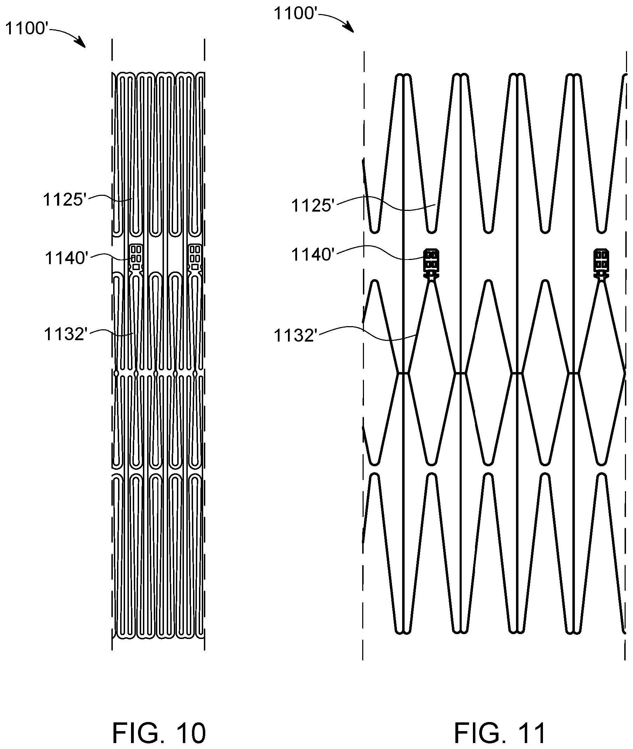

Fig. 10 illustrates the cut pattern of a portion of a stent 1100' that is identical to stent 1100, with the exception that CAFs 1140' are included. Fig. 11 illustrates the same cut pattern as Fig. 10 but after partial expansion. The CAFs 1140' may be identical to CAFs 140 in structure. If the prosthetic heart valve that incorporates stent 1100' included three prosthetic leaflets 500, stent 1100' preferably includes three CAFs 1140' equally spaced around the circumference of the stent 1100'. For example, stent 1100' may include nine center cells 1132', and every third cell 1132' includes a CAF 1140' extending from the outflow apex of that cell. Thus, the CAFs 1140' have a first end coupled to a cell 1132' and a second free end that is generally aligned with a peak of a "V"-shaped strut 1125'. Stent 1100', after being formed, may be shape set to have a similar or identical shape to stent 1100 as shown in Fig. 8.

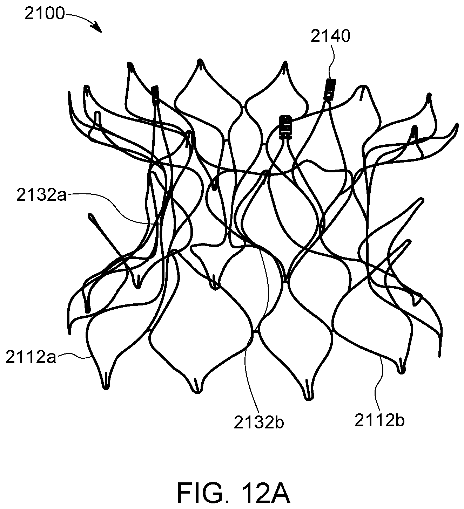

Fig. 12A is a perspective view of a stent 2100 for use with a prosthetic heart valve like prosthetic heart valve 10. Stent 2100 may be similar or identical to stent 100 in most aspects, and thus only the differences are described below. In particular, the ventricular side of stent 2100 may be similar or identical to stent 100 and is not described further. The atrial end of the stent 2100 (towards the bottom in the view of Fig. 12A) may include additional features not found in stent 100. For example, rather than a generally uniform row of atrial cells 112, stent 2100 may include alternating types of diamond-shaped atrial cells. In particular, connected atrial cells 2112a may include an outflow apex that is coupled to an inflow apex of a connected center cell 2132a or to an inflow apex of a free center cell 2132b which includes a CAF 2140. Between each connected atrial cell 2112a may be a free atrial cell 2112b. Each free atrial cell 2112b may have a free outflow apex, with the outflow portion of the free atrial cell 2112b shape-set to hook radially outwardly. With this embodiment, the outwardly hooking outflow portion of the free atrial cell 2112b helps to press the outer fabric (*e.g.,* first fabric 200) radially outwardly and into contact with the native valve annulus to enhance sealing.

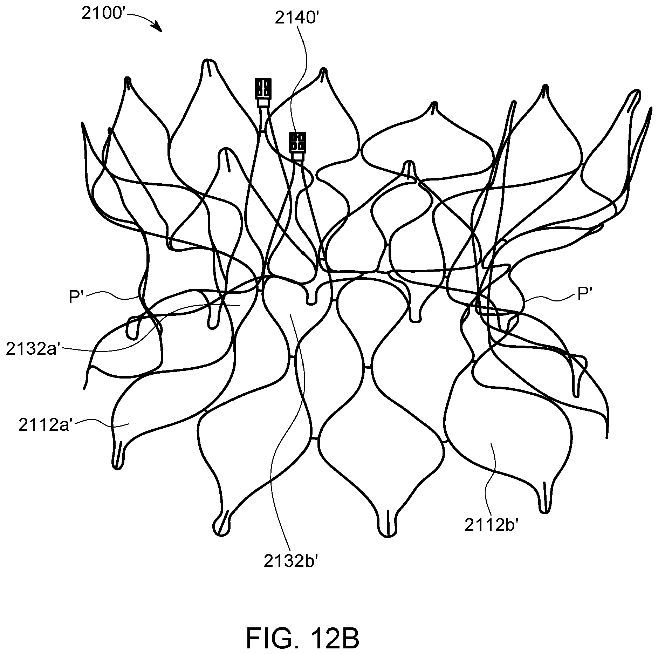

Fig. 12B is a perspective view of a stent 2100' for use with a prosthetic heart valve like prosthetic heart valve 10. Stent 2100' may be similar or identical to stent 100 in most aspects, and thus only the differences are described below. Stent 2100' may also be similar to stent 2100, with the main difference being the stent configuration to provide for outward pressing of the outer fabric into the native valve annulus. The ventricular side of stent 2100' may be similar or identical to stent 100 and is not described further. The atrial end of the stent 2100' (towards the bottom in the view of Fig. 12B) may include additional features not found in stent 100. For example, rather than a generally uniform row of atrial cells 112, stent 2100' may include alternating types of diamond-shaped atrial cells. In particular, connected atrial cells 2112a' may include an outflow apex that is coupled to an inflow apex of a connected center cell 2132a' or to an inflow apex of a free center cell 2132b' which includes a CAF 2140'. Between each series (e.g., series of three) connected atrial cell 2112a' may be a protruding atrial cell 2112b'. Each protruding atrial cell 2112b' may be coupled to a center cell and shape-set to create an outward bump or protrusion P' near the central waisted portion of the stent 2100'. Similar to stent 2100, in this embodiment, the outwardly protruding portion of the free atrial cell 2112b' and/or adjacent central cell helps to press the outer fabric (*e.g.,* first fabric 200) radially outwardly and into contact with the native valve annulus to enhance sealing.

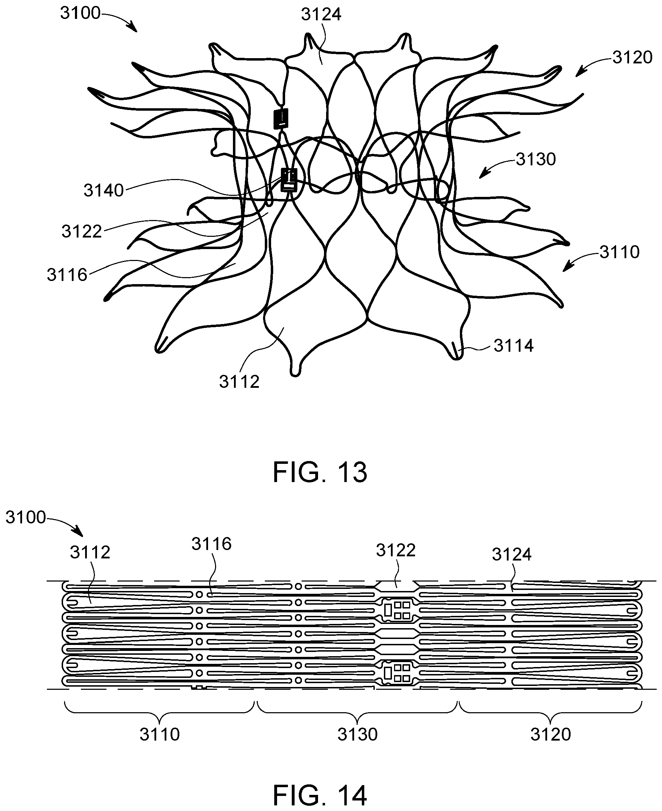

Fig. 13 is a perspective view of a stent 3100 according to another embodiment of the disclosure. The stent 3100 may be used with a prosthetic heart valve like that of prosthetic heart valve 10, with the main difference between replacing stent 100 with stent 3100. Fig. 14 illustrates a portion of a cut pattern of stent 3100. Stent 3100 may be formed of any of the materials described in connection with stent 100 and using any of the methods described in connection with stent 100. Because the only difference between stent 100 and stent 3100 is the stent pattern, only the stent pattern is described below. One of the main differences between stent 100 and stent 3100 is that stent 3100 includes non-cantilevered CAFs 3140.

As with stent 100, stent 3100 may include an atrial disk 3110, a ventricular disk 3120, and a center portion 3130. The atrial disk 3110 may include a row of diamond-shaped atrial cells 3112, and the ventricular disk 3120 may include a row of diamond-shaped ventricular cells 3124. In this example, each row includes twelve cells. The free apices of the atrial cells 3112 may include pins 3114 similar to pins 3114, and the free apices of the ventricular cells 3124 may also include pins 3114. In some embodiments, only one disk or neither disk may include pins 3114, and if pins 3114 are included, they may be in every cell in the row, every other cell, every third cell, *etc.*

The center portion 3130 may be formed in part by atrial transition cells 3116, and in part by ventricular transition cells 3122. Each of these rows of cells may be diamond-shaped and each row may include twelve cells. The atrial transition cells 3116 may be positioned directly adjacent to the atrial cells 3112, and the ventricular transition cells 3122 may be positioned directly adjacent to the ventricular cells 3124. The CAFs 3140 may be positioned between adjacent pairs of the ventricular transition cells 3122, between an atrial transition cell 3116 and a ventricular cell 3124. Compared to stent 100, which includes cantilevered CAFs 140, the fully integrated CAFs 3140 of stent 3100 may require a shorter leaflet height, which may be less preferred in some circumstances.

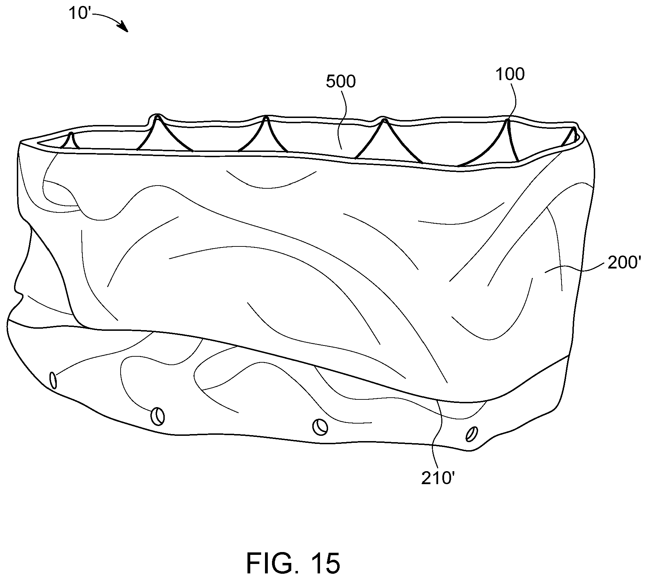

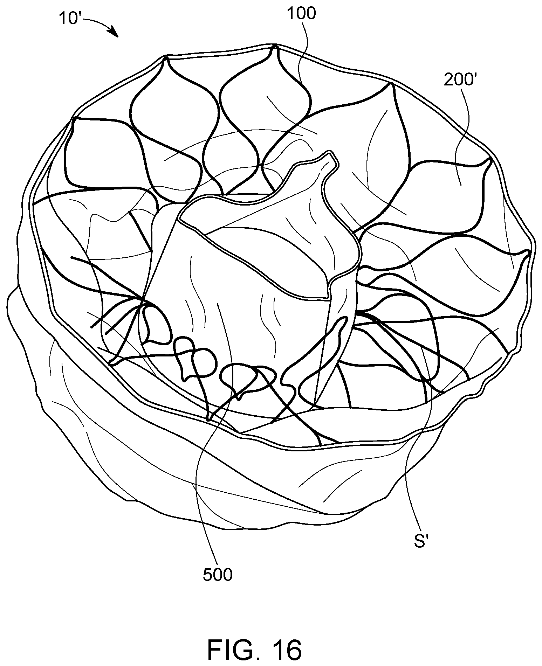

Fig. 15 is a side view of a prosthetic heart valve 10' according to another aspect of the disclosure. Fig. 16 is a perspective view of prosthetic heart valve 10' showing primarily the outflow or ventricular end of the prosthetic heart valve 10'. Prosthetic heart valve 10' may include stent 100 and prosthetic leaflets 500, with the main difference between prosthetic heart valve 10 and 10' being the outer fabric. Prosthetic heart valve 10' includes an outer fabric 200' that is not configured to significantly stretch but rather is configured for sealing. For example, outer fabric 200' may be formed of a tightly woven synthetic fabric (including any of the materials described in connection with the first fabric 200). In order to ensure that the outer fabric 200' does not inhibit the ability of the stent 100 to collapse and expand, the top-to-bottom (or inflow-to-outflow) length of the outer fabric 200' is significantly longer than the top-to-bottom length of the stent 100 when the stent 100 is in the expanded condition, but not significantly longer than the stent 100 when the stent 100 is in the collapsed condition. To achieve this configuration, the inflow end of the outer fabric 200' may be directly coupled to the inflow end of the stent 100, the outflow end of the outer fabric 200' may be directly coupled to the outflow end of the stent 100, and a middle portion of the outer fabric 200' between the ends may be folded over or pleated. For example, in the embodiment shown in Figs. 15-16, a single pleat 210' may be formed in the outer fabric 200', with the pleat 210' unfolding when the stent 100 is collapsed. Although one pleat 210' is shown, it should be understood that more pleats may be provided. In some embodiments, the pleat 210' may be enforced via heating (e.g., via ironing) to help force the outer fabric 200' to preferentially form the pleat 210'. When using outer fabric 200', it may be important to keep the outer fabric 200' generally centered around the native valve annulus, which may be difficult given the large length of the outer fabric 200' and the outer fabric 200' only being connected to the inflow and outflow ends of the stent 100. In other words, the outer fabric 200' may tend to billow out of position and into the right atrium during deployment of the prosthetic heart valve 10'. In order to counteract that tendency, a plurality of connections, shown as sutures S' in Fig. 16, may extend radially inwardly to connect the outer fabric 200' to the stent 100, including to the center portion 130 of the stent 100. Although sutures S' are shown as the connections, other types of connections may be suitable. These sutures S' provide some limits against the outer fabric 200' moving away from the stent 100 during deployment of the prosthetic heart valve 10', but do not otherwise limit the ability of the stent 100 to collapse and expand. Although outer fabric 200' is shown in connection with stent 100, it should be understood that outer fabric 200' may be used with other stents, including any of the other stents described herein. In some embodiments, radiopaque fibers may be woven into the outer fabric 200' to allow for visualization of the outer fabric 200', for example to confirm the desired positioning of the outer fabric 200' relative to the stent 100 and/or to the native valve annulus.

In the embodiment described above, the anchoring of the prosthetic heart valves 10, 10' in the native valve annulus relied primarily on the atrial disks extending onto the atrial side of the valve annulus and the ventricular disks extending onto the ventricular side of the valve annulus, with the disks being large enough to prevent the prosthetic heart valve from dislodging from the native valve annulus. In some embodiments, as described below, additional features may be provided to enhance the anchoring. It should be understood that the enhanced anchoring features described below may be applied to any of the stents described herein.

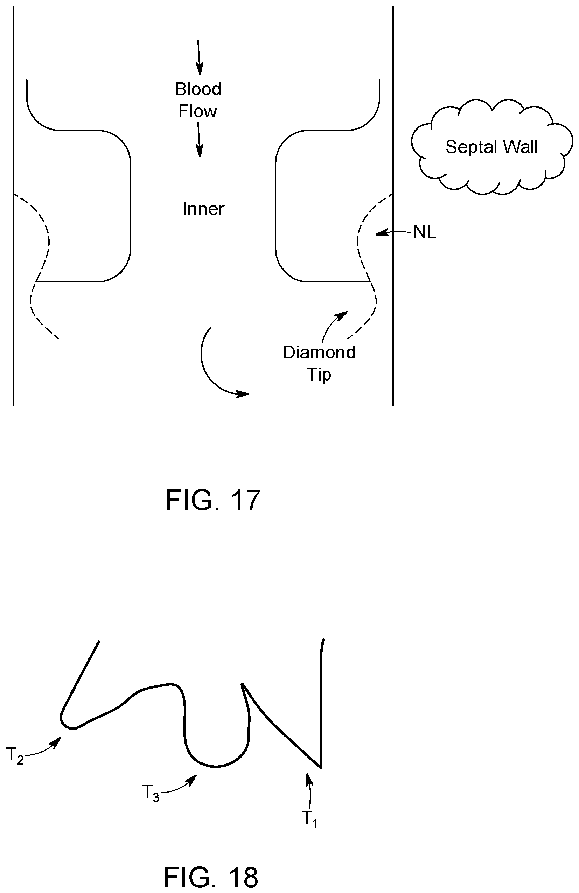

Fig. 17 is a highly schematic drawing of any of the stents described above deployed within a native tricuspid valve annulus. The native tricuspid valve annulus may have a substantially flat septal wall, which may make anchoring slightly more difficult compared to the native mitral valve (although the lower pressures in the tricuspid valve may tend to make anchoring easier compared to the native mitral valve). One way to enhance anchoring is to provide features on the ventricular row of cells that help to dig into the native tissue. Although such features could be provided on both sides, pressures in the right ventricle are higher than those in the right atrium, meaning that the main concern is the prosthetic heart valve migrating into the right atrium. Thus, it may be suitable to provide these anchoring features only on the ventricular side. In the illustrated embodiment, when the stent is deployed, the ventricular disk expands so that it is perpendicular (or nearly perpendicular) to the septal wall, meaning that the free tips of the ventricular cells may "dig in" to tissue, such as the native leaflets NL, to enhance anchoring. As shown in Fig. 18, the outflow tips of the diamond-shaped ventricular cells may be formed with varying geometries, including a sharp tip T1, a medium tip T2, and a soft or blunt tip T3 which may each provide different levels of "digging in" to tissue. As should be understood, the sharper points of sharp tip T1 will tend to dig into native tissue more, while the softer blunted shape of soft tip T3 will tend to dig into native tissue less (or not at all). It should be understood that the prosthetic leaflets and outer fabric are omitted from Fig .17.

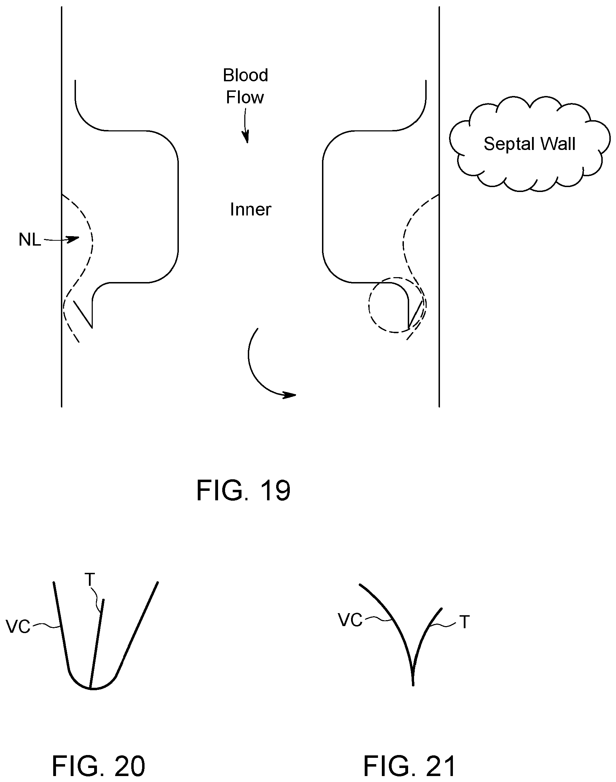

Fig. 19 is a highly schematic drawing of any of the stents described above deployed within a native tricuspid valve annulus with alternate ventricular tip configurations than shown in Figs. 17-18. For example, the stent shown in Fig. 19 has ventricular cells that flare so that they are closer to parallel than perpendicular to the septal wall. However, the ventricular cells VC are formed with tines T extending in the inflow direction from an outflow apex of the cells, as best shown in the front view of Fig. 20. As shown in the side view of Fig. 21, in the deployed condition of the stent, the tines may form an angle of between about 10 degrees and about 20 degrees, including about 15 degrees, relative to the rest of the outflow end of the ventricular cells VC. With this embodiment, as the prosthetic heart valve is deployed in the native tricuspid valve annulus, the tines T flare outwardly relative to the ventricular cells VC in which they are nested, providing a sharp free end that may dig into the native tissue, such as the native leaflets NL as shown in Fig. 19, to enhance anchoring against migration of the prosthetic heart valve into the right atrium. In this embodiment, it may be preferable for the tines T to only extend a short distance to avoid piercing through the relatively thin medial / free ventricular wall.

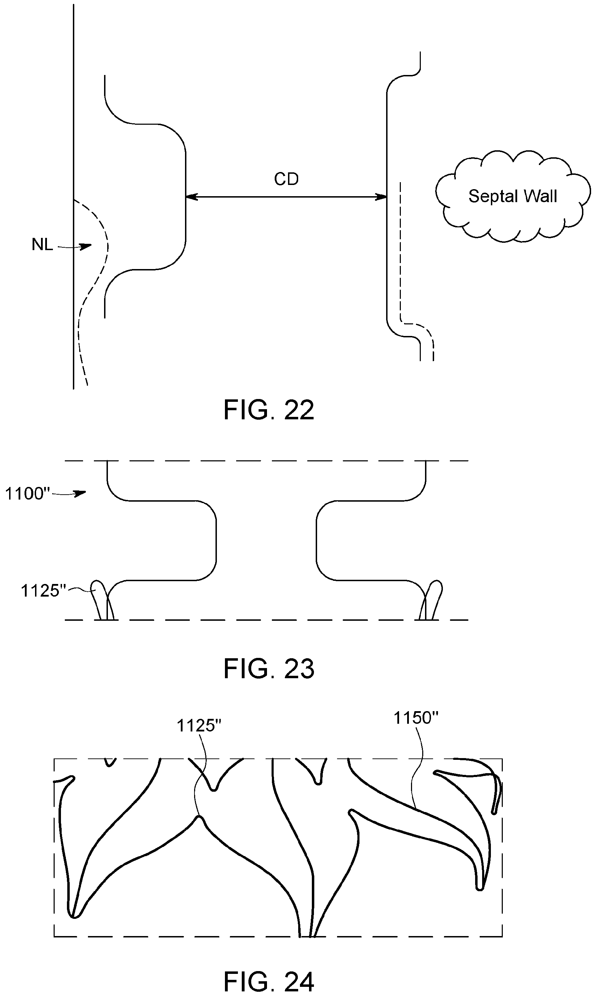

In another embodiment, as shown in Fig. 22, the stent may be deployed in an asymmetric manner in which the stent tends to flatten out against the septal wall, while maintaining a pronounced waisted portion opposite the septal wall. In this embodiment, the central portion of the stent may still have a central diameter CD that is between about 27 mm and about 31 mm, including about 29 mm. This asymmetric shape may be created via shape-setting (*e.g.*, via heat treatment), and when viewed from above the shape may appear as eccentric circles. The shape of the cells may also be non-uniform (*e.g.*, lopsided) to account for different foreshortening requirements of the asymmetric stent. In other embodiments, this type of deformation may be achieved by forming the frame as a soft frame (*e.g.,* a frame with lower-than-typical stiffness).

In another embodiment, the prosthetic heart valve may include a stent with a similar configuration as stent 1100 shown in Fig. 8. However, the "V"-shaped struts 1125" of the ventricular disk may be shape-set so that the tips of those "V"-shaped struts, which are generally centered between adjacent vertical struts 1150", flare radially outwardly to present a pointed structure, as shown in Fig. 23. The enlarged view of Fig. 24 does not show the outward radial flare of the "V"-shaped struts 1125' but is provided to help understand where the radial outward flare would exist. As with the other embodiments described above, upon deployment of stent 1100", the tips of the "V"-shaped struts 1125" would flare radially outwardly to "dig in" to the native tissue in order to provide enhanced anchoring.

For the various embodiments described above, a significant portion of the anchoring of the prosthetic heart valve within the native valve annulus may rely on the atrial disk and the ventricular disk "sandwiching" or otherwise clamping the native valve annulus. This may result in a low susceptibility of the prosthetic heart valve to significantly deform (*e.g.,* to ovalize to a significant degree after implantation).

In an exemplary use of the prosthetic heart valves described herein, a prosthetic heart valve may begin in the expanded condition prior to implantation into a patient. As described above, the prosthetic heart valve may include a single monolithic or unitary stent with an outer fabric on the stent. When the prosthetic heart valve is in the expanded condition, the outer cuff may have a height that is about equal to the height of the stent of the prosthetic heart valve (if the outer cuff is formed to significantly stretch), or the outer cuff may have a height that is significantly larger than the height of the expanded stent (if the outer cuff is formed without significant stretching capabilities). The prosthetic heart valve may be drawn or otherwise forced into a delivery catheter, the prosthetic heart valve transitioning into the collapsed condition as it moves into the delivery catheter. Preferably, the outer diameter of the catheter of the delivery device has a size of 30 French (10 mm) or smaller, including 28 French (9.33 mm) or 24 French (8 mm). As the prosthetic heart valve is drawn into the catheter of the delivery device, the maximum loading force required may be about 45 lbf (about 200 N) for a 24 French (8 mm) inner diameter catheter, preferably as low 30 lbf (about 133 N ) or lower, and about 28 lbf (about 125 N) or lower for a 28 French (9.3 mm) inner diameter catheter. These maximum required forces may be small enough to transition the prosthetic heart valve to the collapsed condition without damaging the prosthetic heart valve. With the prosthetic heart valve successfully collapsed in the small diameter delivery device catheter, the delivery device may be introduced into the patient, for example through the femoral vein, and navigated to the target site, for example the native tricuspid valve. Upon reaching the target site, the prosthetic heart valve may be deployed from the delivery device catheter, for example by retracting the delivery device catheter relative to the prosthetic heart valve. As the constraint on the prosthetic heart valve is removed, the prosthetic heart valve will naturally begin to expand as the stent tends to return to its preset shape. Preferably, the ventricular disk of the prosthetic heart valve is released first within the ventricle (*e.g.,* the right ventricle). As the ventricular disk expands, the ventricular disk may create an anchor point on the ventricular side of the native annulus. If additional anchoring features are included, such as sharp-tipped ventricular cells, those features may tend to dig into or otherwise enhance the anchoring upon expansion of the ventricular disk. As deployment continues, the center portion of the stent of the prosthetic heart valve will remain generally centered within the native valve annulus, with little or no direct contact with the native valve annulus. As the atrial side of the prosthetic heart valve deploys, the atrial disk of the stent will expand and create another anchor point on the atrial side of the native valve annulus. Depending on the type of outer fabric used, the outer fabric will either shorten (*e.g*., relax from the prior stretching if a stretching fabric is used) or will tend to bunch up (*e.g.,* form a pleat or otherwise bunch) around the central portion of the stent. This outer fabric may provide contact with and sealing with the native valve annulus. Thus, as described above, a small delivery device may be used, despite the requirement of coverage of a large native valve annulus area, and without losing any sealing capabilities despite using only a single stent with a small center portion housing the prosthetic leaflets.

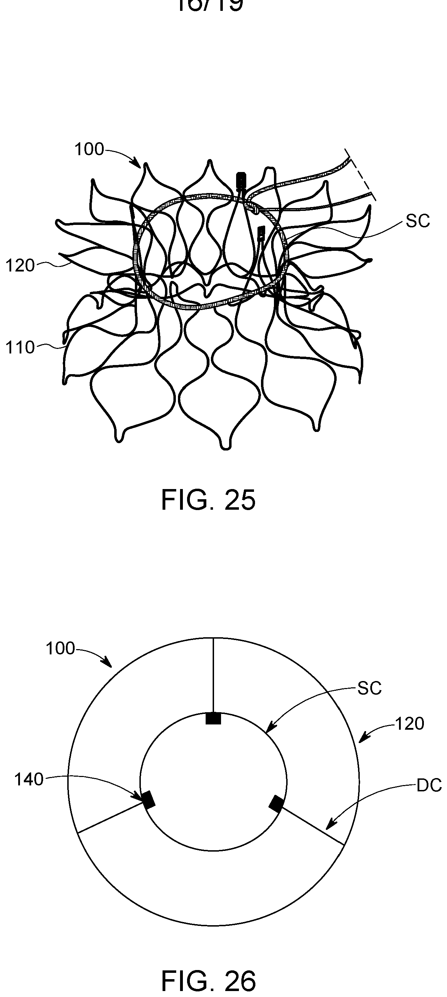

Some of the stents described above include cantilevered commissures attachment features. For example, referring briefly back to Fig. 6, CAFs 140 are coupled to the stent 100 on one side, but not coupled to any stent structure on the opposite side. While this cantilevered or free configuration of CAFs 140 may provide certain benefits, it may present possible drawbacks as well. For example, in a typical deployment of prosthetic heart valve 10, the ventricular disk 120 is the first part of the stent 100 to become free of the constraints of the overlying delivery sheath, with the CAFs 140 following shortly thereafter. In this early stage of deployment, a significant part of the stent 100 may remain collapsed in the delivery device as the ventricular disk 120 and CAFs 140 become free and begin to self-expand. This particular configuration may result in the CAFs 140 beginning to splay radially outwardly, to a degree that is larger than the spacing of the CAFs 140 when no forces are applied to the stent 100. Stated otherwise, while the delivery device sheath is still pinching the atrial end of the stent 100, the ventricular end (including CAFs 140) may tend to overexpand temporarily during deployment. This same splaying may occur as the prosthetic heart valve 10 is being loaded into the delivery device for delivery, particularly while the atrial portion of the prosthetic heart valve 10 has been loaded into the delivery device but while the ventricular end is still free. Because the prosthetic leaflets 500 are directly attached to the CAFs 140, it is generally not desirable for the CAFs 140 to be spaced, even temporarily, a greater distance from the longitudinal center of the stent 100 than would be expected in the absence of applied forces to the stent 100. The prosthetic leaflets 100 are more susceptible to damage than the stent 100, and outward splaying of the CAFs 140 (even temporarily) may put stress on the prosthetic leaflets 500 that could tear or otherwise damage the prosthetic leaflets 500. In order to prevent the CAFs 140 splaying outwardly beyond a preset limit, a splay control SC device may be provided on stent 100. Figs. 25-26 illustrate a splay control SC in the form of a circumferentially extending thread, wire, suture, or similar structure that wraps around the CAFs 140. The splay control SC may be sutured to the stent 100 at or adjacent the CAFs 140, and preferably follows around the outer diameter of the CAFs 140 to avoid interfering with closure of the prosthetic leaflets 500. If the splay control SC is a suture or thread, it may have a length that is about equal to the circumference of an imaginary circle which would pass through all three CAFs 140 when the stent 100 is in an expanded condition in the absence of applied forces (*e.g.,* sitting expanded on a table). As the prosthetic heart valve begins to exit the catheter of the delivery sheath, or just prior to the ventricular end of the prosthetic heart valve 10 being loaded into the catheter of the delivery sheath, the splay control SC will tend to limit the ability for the CAFs 140 to "over"-expand and splay outwardly so much as to damage the prosthetic leaflets 500, as the splay control SC will limit the ability of the CAFs 140 to splay beyond the preset limit.

Another potential drawback of the cantilevered or "hanging" CAFs 140 of stent 100 is for over-deflection of the CAFs 140 during ventricular systole. As the ventricle contracts and the prosthetic leaflets 500 close, the prosthetic leaflets 500 tend to pull the CAFs 140 radially inwardly as the systolic pressures force the leaflets close. Typically, a small amount of deflection (for example about 1 mm) is desirable because deflection of the CAFs 140 will reduce stress on the prosthetic leaflets 500 as the prosthetic leaflets close. However, with the high pressures of ventricular systole and the CAFs 140 being cantilevered, there may be a risk that the CAFs 140 deflect too far radially inwardly. If the CAFs 140 deflect too far radially inwardly, two distinct issues may arise. First, if the prosthetic leaflets 500 are causing the CAFs 140 to deflect inwardly, the deflecting section of the stent - which is typically formed of nitinol - may experience a large moment arm causing strain on the pivot point, which could result in fracturing of the stent at or near the CAF 140, which in turn may result in the inability of the prosthetic leaflets 500 to properly coapt. Second, if the leaflets 500 move too far inwardly during ventricular systole, the prosthetic leaflets 500 may coapt to a degree where they begin to pinwheel, fold, or leak due to the excess leaflet tissue at the point of coaptation. This may result in the loss of a firm seal between the prosthetic leaflets 500 during ventricular systole. As shown in Fig. 26, a plurality of deflection controls DC may be provided to prevent the CAFs 140 from deflecting too far inwardly during ventricular systole. In the illustrated example, the deflection controls DC are sutures, threads, wires, or similar structures that have a first end coupled to the CAF 140 (or a portion of the stent 100 adjacent the CAF 140), and a second end coupled to the ventricular disk 120. During ventricular systole, the CAFs 140 may be prevented from deflecting inwardly a significant distance because of tension on the CAFs 140 provided by the deflection controls DC.

Much of the above disclosure focuses on the use of a prosthetic atrioventricular valve that uses a single stent with an outer sealing and/or stretching fabric to allow for the prosthetic heart valve to fit within a catheter having a small inner diameter. However, some of the outer fabrics described above may still be useful for a double-stent valve (e.g., a prosthetic mitral valve). Even though a double-stent prosthetic heart valve will be unlikely to collapse to a size as small as a single-stent prosthetic heart valve, certain outer fabric configurations may allow for a prosthetic heart valve to achieve a smaller collapsed profile, whether those outer fabric configurations are applied to a prosthetic heart valve with a single frame or with a double frame. And it is typically desirable to achieve a lower collapsed profile size, whether the prosthetic heart valve includes a single frame or a double frame. Certain outer fabric configurations are described below that may allow a prosthetic heart valve to achieve a smaller collapse profile compared to traditional sealing fabrics, and while these outer fabric configurations are shown and described in the context of a double-frame prosthetic heart valve, it should be understood that the concepts apply to single-frame prosthetic heart valves, including the stents described above.

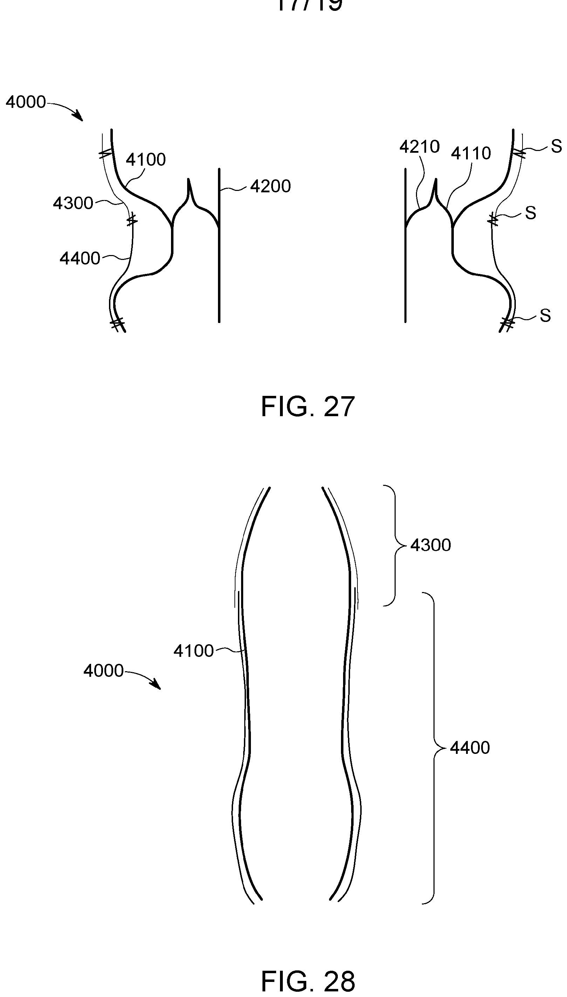

Fig. 27 is a cross-section of a prosthetic heart valve 4000 according to another aspect of the disclosure. Unlike other prosthetic heart valves shown above, prosthetic heart valve 4000 includes an outer frame or stent 4100 and an inner frame or stent 4200. Although not shown in Fig. 27, a set (preferably of three) prosthetic leaflets would be mounted to the generally cylindrical inner frame 4200. The outer frame 4100 may have a plurality of inwardly extending connecting arms 4110 that are connected to a corresponding plurality of outwardly extending connecting arms 4210 of the inner frame 4200 in order to couple the inner and outer frames. Suitable double frame designs are described in greater detail in U.S. Patent Application Publication No. 2022/0087814. In certain prior art double frame prosthetic heart valves having a similar double frame design as shown in Fig. 27, a non-extensible sealing skirt may be attached along the contours of the outer frame 4100 to help provide sealing. However, because those prior art sealing skirts are non-extensible, they typically need to have a length that is about equal to or larger than the length of the outer frame 4100 when the outer frame is collapsed. This may require an overall larger amount of material for the outer skirt and create more bulk, increasing the overall profile of the prosthetic heart valve when collapsed. Figs. 27-28 illustrate one option to reduce the total overall outer fabric that has similarities to the embodiment shown in Figs. 1-5. In particular, a relatively short length of a first outer fabric 4300 is coupled to the atrial disk of the outer frame 4100, for example via sutures S or ultrasonic welding. The first outer fabric 4300 is a sealing fabric that is not stretchable or extensible, for example a woven fabric of any suitable material, such as PET. A second fabric 4400 may be coupled to the ventricular disk of the outer frame 4100, for example via sutures S. The second fabric 4400 may be longer than the first fabric 4300, and attached to the first fabric 4300 by any suitable mechanism, including by sutures S. The second fabric 4400 may be formed as a highly stretchable or extensible fabric, such as a knit fabric of any suitable material, for example PET. As shown in Fig. 27, there may be only minimal overlap between the first fabric 4300 and the second fabric 4400.

Fig. 28 illustrates prosthetic heart valve 4000 in a collapsed condition, omitting the inner frame 4200 and prosthetic leaflets for purposes of clarity. By comparing Fig. 27 to Fig. 28, it can be seen that the first fabric 4300 does not stretch any significant amount upon collapsing, whereas the second fabric 4400 significantly extends. As described with other embodiments above, the goal of the outer fabric is generally to ensure proper sealing with the native valve annulus, while maintaining the minimal amount of bulk and while not hindering the ability of the prosthetic heart valve frame(s) to collapse and expand. As best shown in Fig. 27, the length of the combined outer skirt when the prosthetic heart valve 4000 is expanded is significantly smaller than the length of the prosthetic heart valve when the prosthetic heart valve is collapsed. This allows for a smaller amount of total outer fabric which may reduce the overall crimp or collapsed profile of the prosthetic heart valve 4000, allowing for smaller delivery devices to be used. This embodiment may apply to any prosthetic heart valve with a foreshortening frame, whether a single frame (similar to prosthetic heart valve 10 of Figs. 1-5) or a double frame.

Despite the potential benefits of the outer skirt configuration of Figs. 27-28, certain drawbacks may exist. In particular, there is a limit to the depth that the non-extensible first fabric 4300 is able to extend toward the central waist or ventricular side of the outer frame 4100. For example, if the first non-extensible outer fabric 4300 were too long, and the second highly extensible outer fabric 4400 were too short, the prosthetic heart valve 4000 may not be able to transition to the collapsed condition without the combined outer fabric constraining the frame from collapsing. On the other hand, if the first non-extensible outer fabric 4300 were too short, the prosthetic heart valve 4000 may not be able to achieve a proper seal between the patient's ventricle and atrium. Stated differently, the prosthetic heart valve 4000 may not achieve the desired functionality if the outer fabrics were either too short or too long. The embodiments described below have additional features that may mitigate the potential drawback resulting from the competing advantage of the first non-extensible fabric 4300 being either too long or too short. As should be understood, the outer fabric configurations of Figs. 27-32 are shown in the context of a double-frame prosthetic heart valve but would apply with generally equal force to the single-frame prosthetic heart valves described above.

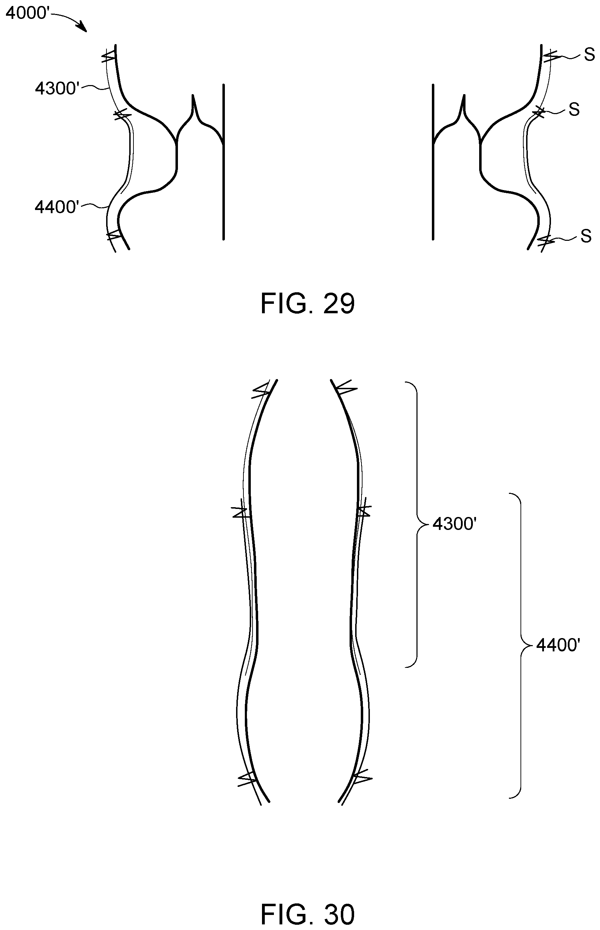

Figs. 29-30 illustrate prosthetic heart valve 4000' in an expanded and collapsed condition, respectively. Other than the configuration of outer fabrics, prosthetic heart valve 4000' may be identical to prosthetic heart valve 4000 and thus components other than the outer fabrics are not described again here. As shown in Figs. 29-30, the first non-extensible sealing fabric 4300' is again coupled to the atrial disk of the outer frame, for example via sutures S, and is also coupled to an end of the second extensible fabric 4400'. However, unlike the embodiment of Figs. 27-28, the first non-extensible sealing fabric 4300' extends an additional distance towards the ventricular end beyond its connection point to the second extensible fabric 4400'. This additional overlap is perhaps best shown by comparing Figs. 28 and 30. The second extensible fabric 4400', as in the prior embodiment, is coupled to the ventricular disk of the outer frame, for example by sutures S. The additional length of the first non-extensible sealing fabric 4300' may help to ensure that the sealing fabric is long enough to create a good seal with the native valve annulus. However, the additional length of the first non-extensible sealing fabric 4300' does not limit the length of the second extensible fabric 4400', meaning that the second extensible fabric 4400' may still be long enough to provide enough stretching so as to not constrain the ability of the prosthetic heart valve 4000' to collapse. The additional length of the first fabric 4300' extending beyond the connection point S to the second fabric 4400' may be either on the inside or outside of the second fabric 4400', but is preferably inside the second fabric 4400' so that the free length of the first fabric 4300' is contained in a defined space and does not, for example, get pushed into the atrium during deployment of the prosthetic heart valve 4400'. In the expanded condition of the prosthetic heart valve 4000', the total length of the first fabric 4300' may be about equal to the total length of the (non-extended) second fabric 4400'. In other embodiments, the total length of the first fabric 4300' may be anywhere from 50% smaller to 100% longer or more than the total length of the (non-extended) second fabric 4400'. In some embodiments, the length of the first fabric 4300' between the connection point S to the second fabric 4400' and the free end of the first fabric 4300' may be about equal to the length of the first fabric 4300' between the connection points S to the atrial disk and the connection point S to the second fabric 4400'. In other embodiments, the free length of the first fabric 4300' may be between 50% smaller and 100% longer or more than the length of the first fabric 4300' between its two connection points S.

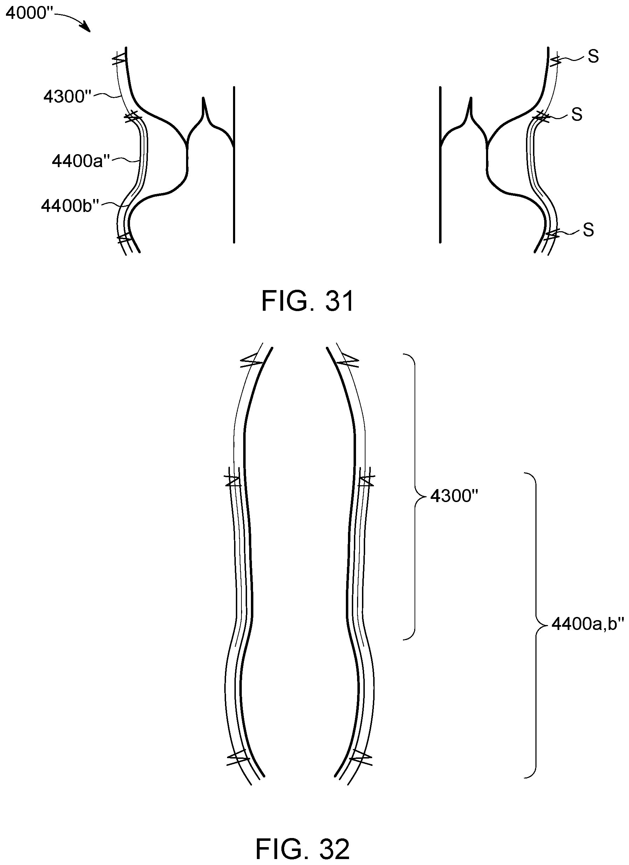

Figs. 31-32 illustrate prosthetic heart valve 4000" in an expanded and collapsed condition, respectively. Other than the configuration of outer fabrics, prosthetic heart valve 4000" may be identical to prosthetic heart valve 4000 and 4000' and thus components other than the outer fabrics are not described again here. The only difference between prosthetic heart valve 4000' and 4000" is that prosthetic heart valve 4000" includes two layers of the extensible second fabric, a first outer layer 4400a" and a second inner layer 4400b". With this configuration, the free length of the first non-extensible sealing fabric 4300" beyond its connection point S to the second fabric layers 4400a", 4400b" is effectively sandwiched between the two second fabric layers 4400a", 4400b". The benefits of this configuration are essentially the same as those described in connection with Figs. 29-30, with the added benefit that the overlapping or free length of the first non-extensible sealing fabric 4300" is more likely to be maintained within the waist region of the outer stent because it is not free to move significantly due to its sandwiched configuration. Because the primary purpose of the first non-extensible sealing fabric 4300" is sealing against the native valve annulus, it is generally preferable for the length of the sealing fabric 4300" to be maintained in a known and desired position between the outer frame (or the only frame if this outer fabric configuration is used with a single frame design) and the native valve annulus, which may be achieved by the configuration shown in Figs. 31-32.

Although the invention herein has been described with reference to particular embodiments, it is to be understood that these embodiments are merely illustrative of the principles and applications of the present invention.

## Claims

1. A prosthetic heart valve (10) for replacing a native atrioventricular valve, the prosthetic heart valve comprising:
a self-expanding stent (100, 1100, 110', 2100, 2100', 3100) formed as a monolithic structure, the stent including an atrial disk (110, 1110, 3110), a ventricular disk (120, 1120, 3120), and a central portion (130, 1130, 3130) extending between the atrial disk and the ventricular disk;
a plurality of prosthetic leaflets (500) directly coupled to the central portion of the stent; and
an outer fabric coupled to the stent, wherein in an implanted condition of the prosthetic heart valve, the atrial disk is sized to contact an atrial side of the native atrioventricular valve, the ventricular disk is sized to contact a ventricular side of the native atrioventricular valve, the central portion is sized to be positioned radially inside the native atrioventricular valve without pressing against the native atrioventricular valve, and the outer fabric is configured to directly contact the native atrioventricular valve,
**characterized in that** the outer fabric includes a first outer fabric (200) and a second outer fabric (300), the first outer fabric being formed as an extensible fabric that is not directly coupled to the central portion of the stent, the second outer fabric being formed as a non-extensible fabric, the second outer fabric including a first portion directly coupled to the atrial disk and a second portion directly coupled to the ventricular disk.

2. The prosthetic heart valve (10) of claim 1, wherein in a collapsed condition of the prosthetic heart valve, the prosthetic heart valve is sized to be received within a delivery catheter for delivery to the patient, the delivery catheter having an inner diameter of between 22 French and 32 French.

3. The prosthetic heart valve (10) of claim 1 or 2, wherein in an expanded condition of the stent (100), the central portion (130) has a diameter, and the ventricular disk (120) has a diameter that is between 1.5 and 2.5 times larger than the diameter of the central portion.

4. The prosthetic heart valve of any preceding claim, wherein a first end of the first outer fabric (200) is coupled to the first portion of the second outer fabric (300) at a first seam (400), and a second end of the first outer fabric is coupled to the second portion of the second outer fabric at a second seam (400).

5. The prosthetic heart valve of any preceding claim, wherein the first seam (400) and the second seam (400) are formed via sutures or ultrasonic welding.

6. The prosthetic heart valve of any preceding claim, wherein as the prosthetic heart valve transitions from an expanded condition to a collapsed condition, the first outer fabric (200) stretches to increase in length by a factor of greater than 1 and less than 3.

7. The prosthetic heart valve of any preceding claim, wherein the stent (100, 2100, 2100', 3100) includes a plurality of cells (112, 116, 132a, 132b, 122, 124a, 124b, 2112a, 2112a', 2112b, 2112b', 2132a, 2132a', 2132b, 2132b', 3112, 3114, 3116, 3122), selected ones of the cells having a free end hooking radially outwardly adjacent the central portion (130, 3130) of the stent in an expanded condition of the stent, the free end configured to press the outer fabric radially outwardly.

8. The prosthetic heart valve (10) of any of claims 1 to 6, wherein the stent (2100') includes a plurality of cells (2112a', 2112b', 2132a', 2132b'), selected ones of the cells having a protrusion (P') extending radially outwardly adjacent the central portion (130) of the stent in an expanded condition of the stent, the protrusion configured to press the outer fabric radially outwardly.

9. The prosthetic heart valve (10) of any preceding claim, wherein the ventricular disk (120) includes a plurality of diamond-shaped ventricular cells that extend substantially parallel to a center longitudinal axis of the stent (100) in an expanded condition of the stent.

10. The prosthetic heart valve (10) of any preceding claim, wherein the non-extensible fabric is a woven fabric, and the extensible fabric is a knitted fabric.

## Patentansprüche

1. Prothetische Herzklappe (10) zum Ersetzen einer nativen Atrioventrikularklappe, wobei die prothetische Herzklappe Folgendes umfasst:
einen selbstexpandierenden Stent (100, 1100, 110', 2100, 2100', 3100), der als eine monolithische Struktur gebildet ist, wobei der Stent eine Atrialscheibe (110, 1110, 3110), eine Ventrikelscheibe (120, 1120, 3120) und einen Mittelabschnitt (130, 1130, 3130) einschließt, der sich zwischen der Atrialscheibe und der Ventrikelscheibe erstreckt;
eine Vielzahl von prothetischen Klappensegeln (500), die direkt mit dem Mittelabschnitt des Stents gekoppelt sind; und
ein äußeres Gewebe, das mit dem Stent gekoppelt ist, wobei im implantierten Zustand der prothetischen Herzklappe die Atrialscheibe so dimensioniert ist, dass sie eine atriale Seite der nativen Atrioventrikularklappe kontaktiert, die Ventrikelscheibe so dimensioniert ist, dass sie eine ventrikuläre Seite der nativen Atrioventrikularklappe kontaktiert, der Mittelabschnitt so dimensioniert ist, dass er radial innerhalb der nativen Atrioventrikularklappe positioniert wird, ohne gegen die native Atrioventrikularklappe zu drücken, und das äußere Gewebe dazu ausgebildet ist, die native Atrioventrikularklappe direkt zu kontaktieren,
**dadurch gekennzeichnet, dass** das äußere Gewebe ein erstes äußeres Gewebe (200) und ein zweites äußeres Gewebe (300) einschließt, wobei das erste äußere Gewebe als ein dehnbares Gewebe gebildet ist, das nicht direkt mit dem Mittelabschnitt des Stents gekoppelt ist, wobei das zweite äußere Gewebe als ein nicht dehnbares Gewebe gebildet ist, wobei das zweite äußere Gewebe einen ersten Abschnitt einschließt, der direkt mit der Atrialscheibe gekoppelt ist, und einen zweiten Abschnitt, der direkt mit der Ventrikelscheibe gekoppelt ist.

2. Prothetische Herzklappe (10) nach Anspruch 1, wobei im kollabierten Zustand der prothetischen Herzklappe die prothetische Herzklappe so dimensioniert ist, dass sie innerhalb eines Einführkatheters zum Einsatz in den Patienten aufgenommen wird, wobei der Einführkatheter einen Innendurchmesser zwischen 22 French und 32 French aufweist.

3. Prothetische Herzklappe (10) nach Anspruch 1 oder 2, wobei im expandierten Zustand des Stents (100) der Mittelabschnitt (130) einen Durchmesser aufweist und die Ventrikelscheibe (120) einen Durchmesser aufweist, der zwischen 1,5-mal und 2,5-mal größer ist als der Durchmesser des Mittelabschnitts.

4. Prothetische Herzklappe nach einem der vorstehenden Ansprüche, wobei ein erstes Ende des ersten äußeren Gewebes (200) an einer ersten Naht (400) mit dem ersten Abschnitt des zweiten äußeren Gewebes (300) gekoppelt ist und ein zweites Ende des ersten äußeren Gewebes an einer zweiten Naht (400) mit dem zweiten Abschnitt des zweiten äußeren Gewebes gekoppelt ist.

5. Prothetische Herzklappe nach einem der vorstehenden Ansprüche, wobei die erste Naht (400) und die zweite Naht (400) durch Suturen oder Ultraschallschweißen gebildet sind.

6. Prothetische Herzklappe nach einem der vorstehenden Ansprüche, wobei, wenn die prothetische Herzklappe von einem expandierten Zustand in einen kollabierten Zustand übergeht, sich das erste äußere Gewebe (200) dehnt, um seine Länge um einen Faktor von größer als 1 und kleiner als 3 zu erhöhen.

7. Prothetische Herzklappe nach einem der vorstehenden Ansprüche, wobei der Stent (100, 2100, 2100', 3100) eine Vielzahl von Zellen (112, 116, 132a, 132b, 122, 124a, 124b, 2112a, 2112a', 2112b, 2112b', 2132a, 2132a', 2132b, 2132b', 3112, 3114, 3116, 3122) einschließt, wobei ausgewählte der Zellen ein freies Ende aufweisen, das im expandierten Zustand des Stents hakenförmig radial nach außen verlaufend benachbart zu dem Mittelabschnitt (130, 3130) des Stents ist, wobei das freie Ende dazu ausgebildet ist, das äußere Gewebe radial nach außen zu drücken.

8. Prothetische Herzklappe (10) nach einem der Ansprüche 1 bis 6, wobei der Stent (2100') eine Vielzahl von Zellen (2112a', 2112b', 2132a', 2132b') einschließt, wobei ausgewählte der Zellen einen Vorsprung (P') aufweisen, der im expandierten Zustand des Stents radial nach außen benachbart zu dem Mittelabschnitt (130) des Stents verläuft, wobei der Vorsprung dazu ausgebildet ist, das äußere Gewebe radial nach außen zu drücken.

9. Prothetische Herzklappe (10) nach einem der vorstehenden Ansprüche, wobei die Ventrikelscheibe (120) eine Vielzahl von rautenförmigen Ventrikelzellen einschließt, die im expandierten Zustand des Stents (100) im Wesentlichen parallel zu einer zentralen Längsachse des Stents verlaufen.

10. Prothetische Herzklappe (10) nach einem der vorstehenden Ansprüche, wobei das nicht dehnbare Gewebe ein Webstoff ist, und das dehnbare Gewebe ein Gestrick ist.

## Revendications

1. Valve cardiaque prothétique (10) pour remplacer une valve atrioventriculaire native, la valve cardiaque prothétique comprenant :
un stent auto-expansible (100, 1100, 110', 2100, 2100', 3100) formé en tant que structure monolithique, le stent incluant un disque auriculaire (110, 1110, 3110), un disque ventriculaire (120, 1120, 3120), et une partie centrale (130, 1130, 3130) s'étendant entre le disque auriculaire et le disque ventriculaire ;
une pluralité de feuillets prothétiques (500) directement couplés à la partie centrale du stent ; et
un tissu externe couplé au stent, dans laquelle dans un état implanté de la valve cardiaque prothétique, le disque auriculaire est dimensionné pour venir en contact avec un côté auriculaire de la valve atrioventriculaire native, le disque ventriculaire est dimensionné pour venir en contact avec un côté ventriculaire de la valve atrioventriculaire native, la partie centrale est dimensionnée pour être positionnée radialement à l'intérieur de la valve atrioventriculaire native sans exercer de pression contre la valve atrioventriculaire native, et le tissu externe est configuré pour venir directement en contact avec la valve atrioventriculaire native,
**caractérisée en ce que** le tissu externe inclut un premier tissu externe (200) et un deuxième tissu externe (300), le premier tissu externe étant formé en tant que tissu extensible qui n'est pas directement couplé à la partie centrale du stent, le deuxième tissu externe étant formé en tant que tissu non extensible, le deuxième tissu externe incluant une première partie directement couplée au disque auriculaire et une deuxième partie directement couplée au disque ventriculaire.

2. Valve cardiaque prothétique (10) selon la revendication 1, dans laquelle dans un état replié de la valve cardiaque prothétique, la valve cardiaque prothétique est dimensionnée pour être reçue à l'intérieur d'un cathéter de pose pour une pose chez le patient, le cathéter de pose présentant un diamètre interne compris entre 22 French et 32 French.

3. Valve cardiaque prothétique (10) selon la revendication 1 ou la revendication 2, dans laquelle dans un état expansé du stent (100), la partie centrale (130) présente un diamètre, et le disque ventriculaire (120) présente un diamètre qui est entre 1,5 et 2,5 fois plus grand que le diamètre de la partie centrale.

4. Valve cardiaque prothétique selon une quelconque revendication précédente, dans laquelle une première extrémité du premier tissu externe (200) est couplée à la première partie du deuxième tissu externe (300) au niveau d'une première couture (400), et une deuxième extrémité du premier tissu externe est couplée à la deuxième partie du deuxième tissu externe au niveau d'une deuxième couture (400).

5. Valve cardiaque prothétique selon une quelconque revendication précédente, dans laquelle la première couture (400) et la deuxième couture (400) sont formées via des sutures ou un soudage par ultrasons.

6. Valve cardiaque prothétique selon une quelconque revendication précédente, dans laquelle, à mesure que la valve cardiaque prothétique passe d'un état expansé à un état replié, le premier tissu externe (200) s'étire pour augmenter en longueur d'un facteur supérieur à 1 et inférieur à 3.

7. Valve cardiaque prothétique selon une quelconque revendication précédente, dans laquelle le stent (100, 2100, 2100', 3100) inclut une pluralité de cellules (112, 116, 132a, 132b, 122, 124a, 124b, 2112a, 2112a', 2112b, 2112b', 2132a, 2132a', 2132b, 2132b', 3112, 3114, 3116, 3122), certaines cellules sélectionnées parmi les cellules présentant une extrémité libre formant crochet radialement vers l'extérieur, de manière adjacente à la partie centrale (130, 3130) du stent dans un état expansé du stent, l'extrémité libre étant configurée pour presser le tissu externe radialement vers l'extérieur.

8. Valve cardiaque prothétique (10) selon l'une quelconque des revendications 1 à 6, dans laquelle le stent (2100') inclut une pluralité de cellules (2112a', 2112b', 2132a', 2132b'), certaines cellules sélectionnées parmi les cellules présentant une saillie (P') s'étendant radialement vers l'extérieur, de manière adjacente à la partie centrale (130) du stent dans un état expansé du stent, la saillie étant configurée pour presser le tissu externe radialement vers l'extérieur.

9. Valve cardiaque prothétique (10) selon une quelconque revendication précédente, dans laquelle le disque ventriculaire (120) inclut une pluralité de cellules ventriculaires en forme de diamant qui s'étendent sensiblement parallèlement à un axe longitudinal central du stent (100) dans un état expansé du stent.

10. Valve cardiaque prothétique (10) selon une quelconque revendication précédente, dans laquelle le tissu non extensible est un tissu tissé, et le tissu extensible est un tissu tricoté.
